(19) Europäisches Patentamt
European Patent Office
Office européen des brevets

(11) EP 1 967 510 A1

(12) EUROPÄISCHE PATENTANMELDUNG

(43) Veröffentlichungstag:
**10.09.2008 Patentblatt 2008/37**

(51) Int Cl.:
***C07C 249/02*** *(2006.01)* ***C07C 251/02*** *(2006.01)*
***C07C 275/00*** *(2006.01)* ***C08G 18/12*** *(2006.01)*

(21) Anmeldenummer: **07103554.7**

(22) Anmeldetag: **06.03.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(71) Anmelder: **Sika Technology AG**
**6340 Baar (CH)**

(72) Erfinder:
- **Schlumpf, Michael**
  **Dietikon, 8953 (CH)**
- **Burckhardt, Urs**
  **Zürich, 8049 (CH)**

(74) Vertreter: **Sika Patent Attorneys**
**c/o Sika Technology AG**
**Corp. IP Dept.**
**Tüffenwies 16**
**Postfach**
**8048 Zürich (CH)**

# (54) Dialdimin, Dialdimin enthaltende Emulsion, sowie zweikomponentige Polyurethanzusammensetzung und deren Verwendungen

(57) Die vorliegende Erfindung betrifft Dialdimine der Formel (I), deren Emulsionen und Verwendungen. Insbesondere lassen sich daraus pastöse Härter- bzw. Beschleunigerkomponenten für Isocyanatgruppen aufweisende Polymere herstellen, die zu einer sehr hohen Frühfestigkeit führen, die ein gutes Fliessverhalten beim Pumpen aufweisen und die ohne VOC-Lösemittel, insbesondere ohne N-Alkylpyrrolidone, formuliert werden können. Diese Komponente lassen sich hervorragend homogen oder inhomogen, insbesondere auch schichtenartig, mit Komponenten, welche Isocyanatgruppen aufweisende Polyurethanpolymere enthalten, vermischen, wobei die so erhaltenen zweikomponentigen Zusammensetzungen rasch aushärten, eine ausserordentlich hohe Frühfestigkeit aufweisen und dabei ohne störende Schwachstellen bleiben.

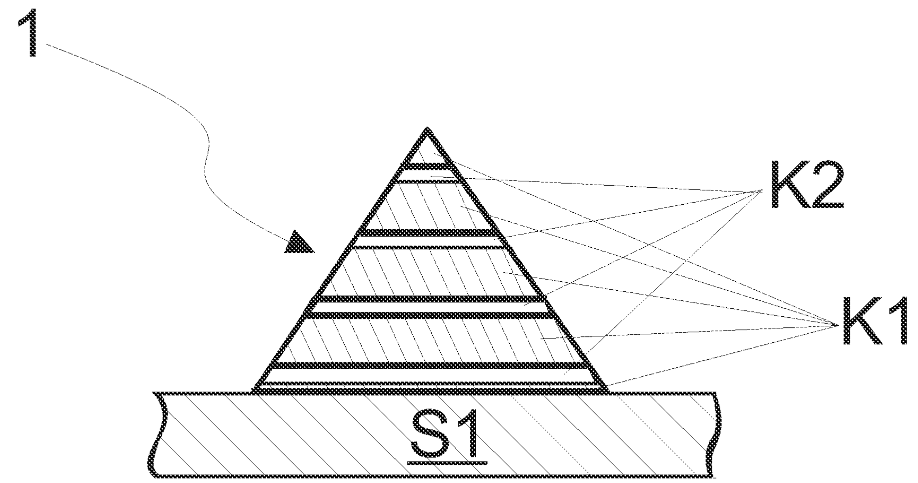


**Figur 1 d)**

EP 1 967 510 A1

## Beschreibung

### Technisches Gebiet

Die Erfindung betrifft das Gebiet der Polyaldimine, der aldiminhaltigen Emulsionen, sowie der zweikomponentigen Polyurethanzusammensetzungen.

### Stand der Technik

Aldimine werden seit langem als blockierte Amine verwendet. Insbesondere werden Polyaldimine als latente Härter in Zusammensetzungen, welche Isocyanatgruppen aufweisende Polymere enthalten und beispielsweise als Kleb- oder Dichtstoffe verwendet werden, eingesetzt. Solche Zusammensetzungen härten beim Kontakt mit Wasser aus, wobei Aldehyde freigesetzt werden. Diese Aldehyde sind meist sehr geruchsintensiv und störend und schränken deshalb die Einsatzmöglichkeiten der Zusammensetzungen stark ein. In WO 2004/013200 A1 werden Polyaldimine enthaltende Polyurethanzusammensetzungen offenbart, die ohne störenden Geruch aushärten.

Einkomponentige Polyurethanzusammensetzungen, welche Isocyanatgruppen aufweisende Polymere enthalten und mit Luftfeuchtigkeit aushärten, werden schon seit langem eingesetzt, beispielsweise als Kleb- oder Dichtstoffe oder als Beschichtungen. Da diese Zusammensetzungen im Allgemeinen langsam aushärten, wurde schon vielfach vorgeschlagen, die Aushärtung, und damit auch den Festigkeitsaufbau, durch Beimischung einer zweiten Komponente, welche Wasser enthält, zu beschleunigen. Solche zweikomponentigen Zusammensetzungen weisen jedoch den grossen Nachteil auf, dass sie zur Bildung von Blasen neigen. Bei einer schichtenartigen Vermischung der beiden Komponenten weisen sie zudem vielfach eine verringerte mechanische Festigkeit auf, insbesondere kurz nach der Vermischung. Die Wasser enthaltende zweite Komponente enthält bei praxistauglichen Systemen meist ein organisches VOC-Lösemittel, insbesondere ein N-Alkylpyrrolidon, was aus arbeitstoxikologischen und arbeitshygienischen Gründen mit grossen Nachteilen verbunden ist.

WO 2005/037885 A1 beschreibt zweikomponentige Polyurethanzusammensetzungen, in welchen zu einer ersten Komponente enthaltend Isocyanatgruppen aufweisende Polyurethanprepolymere eine zweite Komponente, welche neben Wasser geruchsarme Polyaldimine aufweist, zugemischt werden. Diese Zusammensetzungen weisen zwar eine Reduktion von Blasen und eine beschleunigte Aushärtung auf; ihre Aushärtung und ihr Festigkeitsaufbau ist jedoch für viele Anwendungen trotzdem noch zu langsam.

### Darstellung der Erfindung

Die Aufgabe der vorliegenden Erfindung ist es daher, eine zweikomponentige Polyurethanzusammensetzung zur Verfügung zu stellen, welche die Nachteile des Standes der Technik überwindet, und insbesondere über eine hohe Frühfestigkeit verfügt.

Überraschenderweise wurde nun gefunden, dass eine Emulsion, welche auf Dialdiminen der Formel (I) basiert, hervorragend dazu geeignet ist, als Härter- bzw. Beschleunigerkomponente für Isocyanatgruppen aufweisende Polymere, insbesondere für Isocyanatgruppen aufweisende Polyurethanpolymere, verwendet zu werden. Insbesondere lassen sich daraus pastöse Härter- bzw. Beschleunigerkomponenten herstellen, die zu einer sehr hohen Frühfestigkeit führen, die ein gutes Fliessverhalten beim Pumpen aufweisen und die ohne VOC-Lösemittel, insbesondere ohne N-Alkylpyrrolidone, formuliert werden können. Diese Komponenten lassen sich hervorragend homogen oder inhomogen, insbesondere auch schichtenartig, mit Komponenten, welche Isocyanatgruppen aufweisende Polyurethanpolymere enthalten, vermischen, wobei die so erhaltenen zweikomponentigen Zusammensetzungen rasch aushärten, eine ausserordentlich hohe Frühfestigkeit aufweisen und dabei ohne störende Schwachstellen, insbesondere ohne Schwachstellen in oder zwischen den Schichten, vor allem auch dann, wenn die Schichten relativ dick sind, bleiben. Trotz der raschen Aushärtung weisen diese Zusammensetzungen, sowohl bei schichtenartiger als auch bei homogener Vermischung, eine genügend lange Offenzeit auf, die es erlaubt, beispielsweise Verklebungen verlässlich auszuführen.

Die Dialdimine der Formel (I) lassen sich in einfacher Weise aus leicht zugänglichen Dialdiminen, Diisocyanaten und Wasser gezielt herstellen.

Somit betrifft die vorliegende Erfindung Dialdimine der Formel (I) gemäss Anspruch 1, ein Verfahren zu deren Herstellung gemäss Anspruch 11, derartige Dialdimine enthaltende Emulsionen gemäss Anspruch 12 und deren Verwendung als Härterkomponente oder Beschleunigerkomponente für einen Klebstoff, Dichtstoff oder Beschichtungsstoff, welcher Isocyanatgruppen aufweisende Polymere enthält, gemäss Anspruch 14.

Weiterhin betrifft die Erfindung zweikomponentige Zusammensetzungen gemäss Anspruch 15, die daraus erhältlichen vermischten oder teilvermischten Zusammensetzungen gemäss Anspruch 16, die Verwendung der zweikomponentigen Zusammensetzungen als Klebstoff, Dichtstoff oder Beschichtung oder Belag gemäss Anspruch 17, Verfahren zum Verkleben, Abdichten und der Herstellung einer Beschichtung gemäss Anspruch 18, 19 und 20, sowie

die daraus resultierenden Artikel gemäss Anspruch 24.

Bevorzugte Ausführungen der Erfindung sind Gegenstand der abhängigen Ansprüche.

**Wege zur Ausführung der Erfindung**

Die vorliegende Erfindung betrifft Dialdimine der Formel (I).

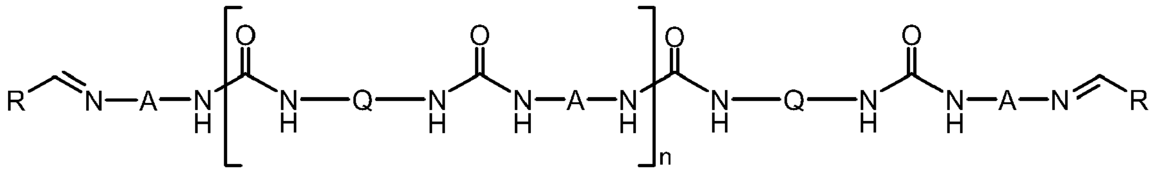

(I)

Hierbei steht R für den Rest eines Aldehyds **ALD** nach Entfernung einer Aldehyd-Gruppe, und A steht für den Rest eines Diamins **DA** mit zwei primären aliphatischen Aminogruppen nach Entfernung der beiden primären Aminogruppen. Die Reste A und R weisen dabei keine Gruppierungen auf, welche in Abwesenheit von Wasser mit Isocyanatgruppen reaktionsfähig sind, insbesondere keine Hydroxylgruppen, keine primären oder sekundären Aminogruppen, keine Mercaptogruppen und keine anderen Gruppen mit aktivem Wasserstoff.

Weiterhin steht Q für den Rest eines Diisocyanates **DI** nach Entfernung beider Isocyanatgruppen, und n steht für 0 oder für eine ganze Zahl von 1 bis 15.

Der Begriff "primäre Aminogruppe" bezeichnet im vorliegenden Dokument eine $NH_2$-Gruppe, die an einen organischen Rest gebunden ist, während der Begriff "sekundäre Aminogruppe" eine NH-Gruppe bezeichnet, die an zwei organische Reste, welche auch gemeinsam Teil eines Rings sein können, gebunden ist.

Als "aliphatische Aminogruppe" wird eine Aminogruppe bezeichnet, die an einen aliphatischen, cycloaliphatischen oder arylaliphatischen Rest gebunden ist. Sie unterscheidet sich damit von einer "aromatischen Aminogruppe", welche direkt an einen aromatischen oder heteroaromatischen Rest gebunden ist, wie beispielsweise in Anilin oder 2-Aminopyridin.

Der Begriff "Polymer" umfasst im vorliegenden Dokument einerseits ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde. Der Begriff umfasst andererseits auch Derivate eines solchen Kollektivs von Makromolekülen aus Polyreaktionen, Verbindungen also, die durch Umsetzungen, wie beispielsweise Additionen oder Substitutionen, von funktionellen Gruppen an vorgegebenen Makromolekülen erhalten wurden und die chemisch einheitlich oder chemisch uneinheitlich sein können. Der Begriff umfasst im Weiteren auch sogenannte Prepolymere, das heisst reaktive oligomere Voraddukte, deren funktionelle Gruppen am Aufbau von Makromolekülen beteiligt sind.

Der Begriff "Polyurethanpolymer" umfasst sämtliche Polymere, welche nach dem sogenannten Diisocyanat-Polyadditions-Verfahren hergestellt werden. Dies schliesst auch solche Polymere ein, die nahezu oder gänzlich frei sind von Urethangruppen. Beispiele für Polyurethanpolymere sind Polyether-Polyurethane, Polyester-Polyurethane, Polyether-Polyharnstoffe, Polyharnstoffe, Polyester-Polyharnstoffe, Polyisocyanurate und Polycarbodiimide.

Als "Raumtemperatur" wird eine Temperatur von 25 °C bezeichnet.

Mit "Poly" beginnende Substanznamen wie Polyaldimin, Polyisocyanat, Polyol oder Polyaldehyd bezeichnen im vorliegenden Dokument Substanzen, die formal zwei oder mehr der in ihrem Namen vorkommenden funktionellen Gruppen pro Molekül enthalten.

Als Aldehyd **ALD** sind grundsätzlich alle bekannten Aldehyde geeignet. Diese Aldehyde können aliphatische, cycloaliphatische, arylaliphatische oder aromatische Aldehyde sein und können Monoaldehyde oder Polyaldehyde sein.

Geeignete aliphatische, cycloaliphatische oder arylaliphatische Aldehyde **ALD** sind beispielsweise Propionaldehyd, Butyraldehyd, Isobutyraldehyd, 2-Methyl-butyraldehyd, 2-Ethyl-butyraldehyd, Valeraldehyd, Isovaleraldehyd, 2-Methyl-valeraldehyd, 2,3-Dimethyl-valeraldehyd, Hexanal, 2-Ethyl-hexanal, Octanal, Isooctanal, Nonanal, Decanal, Undecanal, 2-Methyl-undecanal, Dodecanal, Cyclopropancarboxaldehyd, Cyclopentancarboxaldehyd, Cyclohexylcarboxaldehyd, 1,2,3,6-Tetrahydrobenzaldehyd, Phenylacetaldehyd, 2-Phenylpropionaldehyd (Hydratropaldehyd) und 2-Methyl-3-phenylpropionaldehyd.

Geeignete aromatische Aldehyde **ALD** sind beispielsweise Benzaldehyd, 2- und 3- und 4-Tolualdehyd, 4-Ethyl- und 4-Propyl- und 4-Isopropyl und 4-Butyl-benzaldehyd, 2,4-Dimethylbenzaldehyd, 2,4,5-Trimethylbenzaldehyd, 4-Acetoxybenzaldehyd, 4-Anisaldehyd, 4-Ethoxybenzaldehyd, die isomeren Di-und Trialkoxybenzaldehyde, 2-, 3- und 4-Nitrobenzaldehyd, 2- und 3- und 4-Formylpyridin, 2-Furfuraldehyd, 2-Thiophencarbaldehyd, 1- und 2-Naphthylaldehyd,

3- und 4-Phenyloxy-benzaldehyd; Chinolin-2-carbaldehyd und dessen 3-, 4-, 5-, 6-, 7- und 8-Stellungsisomere, Anthracen-9-carbaldehyd, Phthalaldehyd, Isophthalaldehyd und Terephthalaldehyd.

Es hat sich gezeigt, dass als Aldehyd **ALD** insbesondere solche Aldehyde geeignet sind, welche in $\alpha$-Stellung zur Carbonylgruppe keine C-H-Gruppierung enthalten und deshalb keine tautomeren Enole bilden können.

Der Aldehyd **ALD** stellt somit insbesondere ein Aldehyd der Formel (II-a) oder ein Aldehyd der Formel (II-b) dar.

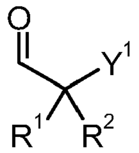

(II-a)

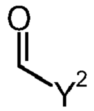

(II-b)

Hierbei stehen $R^1$ und $R^2$ entweder unabhängig voneinander jeweils für einen einwertigen Kohlenwasserstoffrest mit 1 bis 12 C-Atomen, oder zusammen für einen zweiwertigen Kohlenwasserstoffrest mit 4 bis 20 C-Atomen, der Teil eines, gegebenenfalls substituierten, carbocyclischen Rings mit 5 bis 8, bevorzugt 6, C-Atomen, ist;

$Y^1$ steht für einen einwertigen Kohlenwasserstoffrest, welcher gegebenenfalls mindestens ein Heteroatom, insbesondere Sauerstoff in Form von Ether-, Carbonyl- oder Ester-Gruppen, aufweist;
$Y^2$ steht entweder
für eine substituierte oder unsubstituierte Aryl- oder Heteroaryl-Gruppe, welche eine Ringgrösse von 5 bis 8, bevorzugt 6, Atomen aufweist, oder für

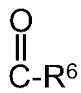 ,

wobei $R^6$ für ein Wasserstoffatom oder für eine Alkoxygruppe steht,
oder für eine substituierte oder unsubstituierte Alkenyl- oder Arylalkenylgruppe mit mindestens 6 C-Atomen.

Geeignete Aldehyde der Formel (II-a) sind beispielsweise 2,2-Dimethyl-propanal (Pivalaldehyd), 2,2-Dimethylbutanal, 2,2-Diethyl-butanal, 1-Methyl-cyclopentancarboxaldehyd, 1-Methyl-cyclohexancarboxaldehyd, sowie die weiter unten beschriebenen Ether und Ester von 2,2-disubstituierten 3-Hydroxypropanalen, -butanalen oder analogen höheren Aldehyden, insbesondere von 2,2-Dimethyl-3-hydroxypropanal.

Geeignete Aldehyde der Formel (II-b) sind beispielsweise die bereits als Aldehyd **ALD** geeignet genannten aromatischen Aldehyde, sowie Glyoxal, Glyoxalsäureester wie Glyoxalsäuremethylester, Zimtaldehyd und substituierte Zimtaldehyde.

Besonders geeignete Dialdimine der Formel (I) sind in einer ersten Ausführungsform solche, deren Reste R die Formel (II) aufweisen.

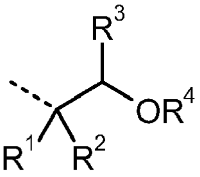

(II)

Hierbei steht $R^3$ für ein Wasserstoffatom oder für eine Alkyl- oder Arylalkylgruppe; und $R^4$ steht für einen, gegebenenfalls Heteroatome enthaltenden, Kohlenwasserstoffrest mit 1 bis 30 C-Atomen.

Besonders geeignete Dialdimine der Formel (I) sind in einer zweiten Ausführungsform solche, deren Reste R

die Formel (III) aufweisen.

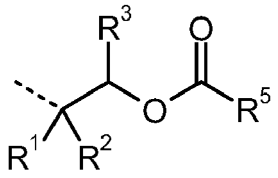 (III)

Hierbei steht $R^5$ entweder für einen linearen oder verzweigten Alkylrest mit 1 bis 30 C-Atomen, gegebenenfalls mit cyclischen Anteilen und gegebenenfalls mit mindestens einem Heteroatom, insbesondere Sauerstoff in Form von Ether- oder Carbonyl- oder Ester-Gruppen, oder für einen einfach oder mehrfach ungesättigten linearen oder verzweigten Kohlenwasserstoffrest mit 1 bis 30 C-Atomen.

Diese Reste R entsprechen der Formel (II), in welchen $R^4$ für den Rest der Formel (III') stehen.

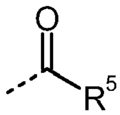 (III')

Bevorzugt sind Dialdimine der Formel (I), welche einen Rest R der Formel (II) oder Formel (III) aufweisen, bei welchem der Rest $R^4$ für einen, gegebenenfalls Heteroatome enthaltenden, Kohlenwasserstoffrest mit 11 bis 30 C-Atomen steht; beziehungsweise bei welchem der Rest $R^5$
für einen linearen oder verzweigten Alkylrest mit 11 bis 30 C-Atomen, gegebenenfalls mit cyclischen Anteilen und gegebenenfalls mit mindestens einem Heteroatom, insbesondere Sauerstoff in Form von Ether- oder Carbonyl- oder Ester-Gruppen, steht,
oder für einen einfach oder mehrfach ungesättigten, linearen oder verzweigten Kohlenwasserstoffrest mit 11 bis 30 C-Atomen steht.

Diese bevorzugten Dialdimine der Formel (I) zeichnen sich dadurch aus, dass sie selbst, beziehungsweise die bei ihrer Hydrolyse entstehenden Aldehyde, geruchsfrei sind. Unter einer "geruchsfreien" Substanz wird in diesem Dokument eine Substanz verstanden, die so geruchsarm ist, dass sie für die meisten menschlichen Individuen nicht riechbar, das heisst mit der Nase nicht wahrnehmbar, ist.

Besonders geeignete Aldehyde der Formel (II-a) stellen einerseits Aldehyde **ALD1** der Formel (II-a'), also Aldehyde **ALD** mit dem Rest R der Formel (II), dar.

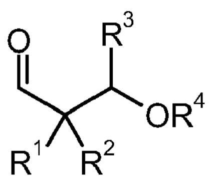 (II-a')

Die Aldehyde **ALD1** der Formel (II-a') stellen Ether von aliphatischen, arylaliphatischen oder cycloaliphatischen 2,2-disubstituierten 3-Hydroxyaldehyden mit Alkoholen oder Phenolen der Formel $R^4$-OH, beispielsweise Fettalkoholen oder Phenol, dar. Geeignete 2,2-disubstituierte 3-Hydroxyaldehyde sind ihrerseits erhältlich aus Aldol-Reaktionen, insbesondere gekreuzten Aldol-Reaktionen, zwischen primären oder sekundären aliphatischen Aldehyden, insbesondere Formaldehyd, und sekundären aliphatischen, sekundären arylaliphatischen oder sekundären cycloaliphatischen Aldehyden, wie beispielsweise 2-Methylbutyraldehyd, 2-Ethylbutyraldehyd, 2-Methylvaleraldehyd, 2-Ethylcapronaldehyd, Cyclopentancarboxaldehyd, Cyclohexancarboxaldehyd, 1,2,3,6-Tetrahydrobenzaldehyd, 2-Methyl-3-phenylpropionaldehyd, 2-Phenylpropionaldehyd (Hydratropaldehyd) oder Diphenylacetaldehyd. Beispiele geeigneter 2,2-disubstituierter 3-Hydroxyaldehyde sind 2,2-Dimethyl-3-hydroxypropanal, 2-Hydroxymethyl-2-methyl-butanal, 2-Hydroxymethyl-2-ethyl-butanal, 2-Hydroxymethyl-2-methyl-pentanal, 2-Hydroxymethyl-2-ethyl-hexanal, 1-Hydroxymethyl-cyclopentancarboxaldehyd, 1-Hydroxymethyl-cyclohexancarboxaldehyd 1-Hydroxymethyl-cyclohex-3-encarbox-aldehyd, 2-Hydroxymethyl-2-methyl-3-phenyl-propanal, 3-Hydroxy-2-methyl-2-phenyl-propanal und 3-Hydroxy-2,2-diphenyl-propanal.

Beispiele für Aldehyde **ALD1** der Formel (II-a') sind 2,2-Dimethyl-3-phenoxy-propanal, 3-Cyclohexyloxy-2,2-dimethyl-propanal, 2,2-Dimethyl-3-(2-ethylhexyloxy)-propanal, 2,2-Dimethyl-3-lauroxy-propanal und 2,2-Dimethyl-3-stearoxy-propanal.

Besonders geeignete Aldehyde der Formel (II-a) stellen andererseits Aldehyde **ALD2** der Formel (II-a''), also Aldehyde **ALD** mit dem Rest R der Formel (III), dar.

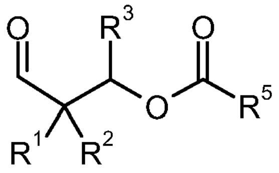

(II-a'')

Die Aldehyde **ALD2** der Formel (II-a'') stellen Ester der bereits beschriebenen 2,2-disubstituierten 3-Hydroxyaldehyde mit geeigneten Carbonsäuren dar.

Beispiele für geeignete Carbonsäuren sind gesättigte aliphatische Carbonsäuren, wie Ameisensäure, Essigsäure, Propionsäure, Buttersäure, Isobuttersäure, Valeriansäure, Capronsäure, 2-Ethyl-capronsäure, Oenanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure; einfach ungesättigte aliphatische Carbonsäuren wie Palmitoleinsäure, Ölsäure, Erucasäure; mehrfach ungesättigte aliphatische Carbonsäuren wie Linolsäure, Linolensäure, Elaeostearinsäure, Arachidonsäure; cycloaliphatische Carbonsäuren wie Cyclohexancarbonsäure; arylaliphatische Carbonsäuren wie Phenylessigsäure; aromatische Carbonsäuren wie Benzoesäure, Naphthoesäure, Toluylsäure, Anissäure; Isomere dieser Säuren; Fettsäuregemische aus der technischen Verseifung von natürlichen Ölen und Fetten wie beispielsweise Rapsöl, Sonnenblumenöl, Leinöl, Ölbaumöl, Kokosnussöl, Ölpalmkernöl und Ölpalmöl; sowie Dicarbonsäuremonoalkyl- und -arylester, wie sie aus der einfachen Veresterung von Dicarbonsäuren wie Bernsteinsäure, Glutarsäure, Adipinsäure, Pimelinsäure, Korksäure, Azelainsäure, Sebacinsäure, 1,12-Dodecandisäure, Maleinsäure, Fumarsäure, Hexahydrophthalsäure, Hexahydroisophthalsäure, Hexahydroterephthalsäure, 3,6,9-Trioxaundecandisäure und ähnliche Derivate von Polyethylenglykol, mit Alkoholen wie Methanol, Ethanol, Propanol, Butanol, höheren Homologen und Isomeren dieser Alkohole erhalten werden.

Bevorzugte Aldehyde **ALD2** der Formel (II-a'') sind 3-Benzoyloxy-2,2-dimethylpropanal, 3-Cyclohexanoyloxy-2,2-dimethylpropanal, 2,2-Dimethyl-3-(2-ethylhexyloxy)-propanal, 2,2-Dimethyl-3-lauroyloxy-propanal, 2,2-Dimethyl-3-myristoyloxypropanal, 2,2-Dimethyl-3-palmitoyloxy-propanal, 2,2-Dimethyl-3-stearoyloxypropanal und, sowie analoge Ester anderer 2,2-disubstituierter 3-Hydroxyaldehyde.

In einer besonders bevorzugten Ausführungsform ist $R^5$ ausgewählt aus der Gruppe bestehend aus Phenyl, Cyclohexyl, 2-Ethylhexyl und den $C_{11}$-, $C_{13}$-, $C_{15}$- und $C_{17}$-Alkylgruppen.

Als Aldehyd **ALD2** der Formel (II-a'') meist bevorzugt ist 2,2-Dimethyl-3-lauroyloxypropanal.

In einer bevorzugten Herstellmethode des Aldehyds **ALD2** der Formel (II-a'') wird ein 2,2-disubstituierter 3-Hydroxyaldehyd, insbesondere 2,2-Dimethyl-3-hydroxypropanal, welcher beispielsweise aus Formaldehyd (oder Paraformaldehyd) und Isobutyraldehyd, gegebenenfalls in situ, hergestellt werden kann, mit einer Carbonsäure zum entsprechenden Ester umgesetzt. Diese Veresterung kann ohne die Verwendung von Lösemitteln nach bekannten Methoden erfolgen, beschrieben beispielsweise in Houben-Weyl, "Methoden der organischen Chemie", Vol. VIII, Seiten 516 - 528.

Die Aldehyde **ALD2** der Formel (II-a'') sind gegenüber den Aldehyden **ALD1** der Formel (II-a') aufgrund ihrer einfachen Herstellbarkeit bevorzugt.

In einer besonders bevorzugten Ausführungsform ist der Aldehyd **ALD** geruchsfrei. Geruchsfreie Aldehyde **ALD** sind einerseits insbesondere Aldehyde **ALD1** der Formel (II-a'), in welchen der Rest $R^4$ für einen Kohlenwasserstoffrest mit 11 bis 30 C-Atomen, der gegebenenfalls Heteroatome enthält, steht.

Andererseits sind geruchsfreie Aldehyde **ALD** insbesondere Aldehyde **ALD2** der Formel (II-a''), in welchen der Rest $R^5$ entweder für eine lineare oder verzweigte Alkylgruppe mit 11 bis 30 Kohlenstoff-Atomen, gegebenenfalls mit cyclischen Anteilen, und gegebenenfalls mit mindestens einem Heteroatom, insbesondere mit mindestens einem Ether-Sauerstoff, oder für eine einfach oder mehrfach ungesättigte lineare oder verzweigte Kohlenwasserstoffkette mit 11 bis 30 Kohlenstoff-Atomen steht.

Beispiele für geruchsfreie Aldehyde **ALD2** der Formel (II-a'') sind Veresterungsprodukte aus den bereits genannten 2,2-disubstituierten 3-Hydroxyaldehyden mit Carbonsäuren wie beispielsweise Laurinsäure, Tridecansäure, Myristinsäure, Pentadecansäure, Palmitinsäure, Margarinsäure, Stearinsäure, Nonadecansäure, Arachinsäure, Palmitoleinsäure, Ölsäure, Erucasäure, Linolsäure, Linolensäure, Elaeostearinsäure, Arachidonsäure, sowie Fettsäuregemische aus der technischen Verseifung von natürlichen Ölen und Fetten, wie beispielsweise Rapsöl, Sonnenblumenöl, Leinöl, Ölbaumöl, Kokosnussöl, Ölpalmkernöl und Ölpalmöl.

Bevorzugte geruchsfreie Aldehyde **ALD2** sind 2,2-Dimethyl-3-lauroyloxypropanal, 2,2-Dimethyl-3-myristoyloxypropanal, 2,2-Dimethyl-3-palmitoyloxypropanal und 2,2-Dimethyl-3-stearoyloxypropanal. Besonders bevorzugt ist 2,2-Dimethyl-3-lauroyloxypropanal.

Das Diamin **DA** mit zwei primären aliphatischen Aminogruppen weist zwei primäre Aminogruppen auf, welche an einen aliphatischen, cycloaliphatischen oder arylaliphatischen Rest gebunden sind.

Als Diamin **DA** geeignet sind aliphatische, cycloaliphatische oder arylaliphatische Diamine, beispielsweise Ethylendiamin, 1,2-Propandiamin, 1,3-Propandiamin, 2-Methyl-1,2-propandiamin, 2,2-Dimethyl-1,3-propandiamin, 1,3-Butandiamin, 1,4-Butandiamin, 1,3-Pentandiamin (DAMP), 1,5-Pentandiamin, 1,5-Diamino-2-methylpentan (MPMD), 1,6-Hexandiamin, 2,5-Dimethyl-1,6-hexandiamin, 2,2,4-Trimethylhexamethylendiamin, 2,4,4-Trimethylhexamethylendiamin, 1,7-Heptandiamin, 1,8-Octandiamin, 1,9-Nonandiamin, 1,10-Decandiamin, 1,11-Undecandiamin, 1,12-Dodecandiamin und Methyl-bis-(3-aminopropyl)amin, 1,2-, 1,3- und 1,4-Diaminocyclohexan, Bis-(4-aminocyclohexyl)-methan, Bis-(4-amino-3-methylcyclohexyl)-methan, Bis-(4-amino-3-ethylcyclohexyl)-methan, Bis-(4-amino-3,5-dimethylcyclohexyl)-methan, 1-Amino-3-aminomethyl-3,5,5-trimethylcyclohexan (= Isophorondiamin oder IPDA), 2- und 4-Methyl-1,3-diaminocyclohexan und Mischungen davon, 1,3-und 1,4-Bis-(aminomethyl)cyclohexan, 1-Cyclohexylamino-3-aminopropan, 2,5(2,6)-Bis-(aminomethyl)-bicyclo[2.2.1]heptan (NBDA, hergestellt von Mitsui Chemicals), 3(4),8 (9)-Bis-(aminomethyl)-tricyclo[5.2.1.0$^{2,6}$]decan, 1,4-Diamino-2,2,6-trimethylcyclohexan (TMCDA), 3,9-Bis-(3-aminopropyl)-2,4,8,10-tetraoxa-spiro[5.5]undecan sowie 1,3- und 1,4-Xylylendiamin.

Als Diamin **DA** besonders geeignet sind Ethergruppen-haltige aliphatische Diamine, beispielsweise Bis-(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin und höhere Oligomere dieser Diamine, sowie Polyoxyalkylen-Diamine. Letztere stellen typischerweise Produkte aus der Aminierung von Polyoxyalkylen-Diolen dar und sind beispielsweise erhältlich unter dem Namen Jeffamine® (von Huntsman Chemicals), unter dem Namen Polyetheramin (von BASF) oder unter dem Namen PC Amine® (von Nitroil). Insbesondere geeignete Polyoxyalkylen-Diamine sind Jeffamine® D-230, Jeffamine® D-400, Jeffamine® D-2000, Jeffamine® D-4000, Jeffamine® XTJ-511, Jeffamine® ED-600, Jeffamine® ED-900, Jeffamine® ED-2003, Jeffamine® XTJ-568, Jeffamine® XTJ-569, Jeffamine® XTJ-523, Jeffamine® XTJ-536, Jeffamine® XTJ-542, Jeffamine® XTJ-559; Polyetheramin D 230, Polyetheramin D 400 und Polyetheramin D 2000, PC Amine® DA 250, PC Amine® DA 400, PC Amine® DA 650 und PC Amine® DA 2000.

Als Diamin **DA** bevorzugt sind Ethergruppen-haltige aliphatische Diamine, insbesondere ein Ethergruppenhaltiges aliphatisches Diamin, welches ausgewählt ist aus der Gruppe bestehend aus Bis-(2-aminoethyl)-ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin und höheren Oligomeren dieser Diamine, sowie Polyoxyalkylen-Diaminen der Formel (V').

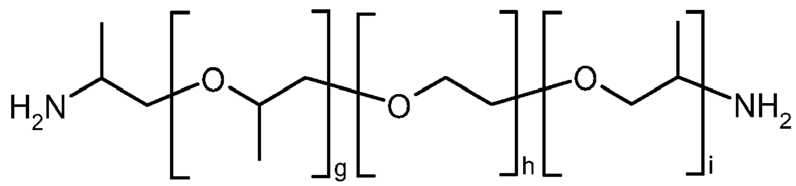

(V')

Hierbei stehen g, h und i jeweils für 0 oder eine ganze Zahl von 1 bis 40, mit der der Massgabe, dass die Summe von g, h und i ≥ 1 ist.

Das Diamin **DA** der Formel (V') weist bevorzugt ein Molekulargewicht zwischen 200 und 5000 g/mol auf.

Beispiele für Polyoxyalkylen-Diamine der Formel (V') sind die im Handel verfügbaren Typen Jeffamine® D-230, Jeffamine® D-400, Jeffamine® D-2000, Jeffamine® D-4000, Jeffamine® XTJ-511, Jeffamine® ED-600, Jeffamine® ED-900 und Jeffamine® ED-2003 (alle von Huntsman Chemical); Polyetheramin D 230, Polyetheramin D 400 und Polyetheramin D 2000 (alle von BASF); sowie PC Amine® DA 250, PC Amine® DA 400, PC Amine® DA 650 und PC Amine® DA 2000 (alle von Nitroil).

Das Diisocyanat **DI** ist entweder ein monomeres Diisocyanat oder ein oligomeres Derivat davon, oder ein Isocyanatgruppen aufweisendes Polyurethanpolymer **P**.

Geeignete monomere Diisocyanate oder oligomere Derivate davon sind beispielsweise 1,6-Hexamethylendiisocyanat (HDI), 2-Methylpenta-methylen-1,5-diisocyanat, 2,2,4- und 2,4,4-Trimethyl-1,6-hexamethylendiisocyanat (TMDI), 1,10-Decamethylendiisocyanat, 1,12-Dodecamethylendiisocyanat, Lysin- und Lysinesterdiisocyanat, Cyclohexan-1,3- diisocyanat, Cyclohexan-1,4-diisocyanat und beliebige Gemische dieser Isomeren, 1-Methyl-2,4- und -2,6-diisocyanatocyclohexan und beliebige Gemische dieser Isomeren (HTDI oder $H_6$TDI), 1-Isocyanato-3,3,5-trimethyl-5-isocyanatomethylcyclohexan (=Isophorondiisocyanat oder IPDI), Perhydro-2,4'-diphenylmethandiisocyanat und Perhydro-4,4'-diphenylmethandiisocyanat (HMDI oder $H_{12}$MDI), 1,4-Diisocyanato-2,2,6-trimethylcyclohexan (TMCDI), 1,3- und 1,4-Bis-(isocyanatomethyl)-cyclohexan, m- und p-Xylylendiisocyanat (m- und p-XDI), m- und p-Tetramethyl-1,3-

und -1,4-xylylendiisocyanat (m- und p-TMXDI), Bis-(1-Isocyanato-1-methylethyl)-naphthalin, 2,4- und 2,6-Toluylendiisocyanat und beliebige Gemische dieser Isomeren (TDI), 4,4'-, 2,4'- und 2,2'-Diphenylmethandiisocyanat und beliebige Gemische dieser Isomeren (MDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanato-diphenyl (TODI), Dianisidindiisocyanat (DADI), Oligomere der vorgenannten Isocyanate, beispielsweise in Form von Uretdionen, Allophanaten und Oxadiazintrionen oder in Form von Addukten mit kurzkettigen Polyolen, sowie beliebige Mischungen der vorgenannten Isocyanate und Oligomeren.

Bevorzugt sind MDI, TDI, HDI und IPDI. Besonders bevorzugt sind MDI und TDI.

Das Diisocyanat **DI** ist bevorzugt ein Isocyanatgruppen aufweisendes Polyurethanpolymer **P**.

Geeignete Isocyanatgruppen aufweisende Polyurethanpolymere **P** weisen zwei Isocyanatgruppen auf und stellen ein Produkt aus der Umsetzung von mindestens einem Diol mit mindestens einem Diisocyanat, insbesondere mit mindestens einem Diisocyanat, welches aus den oben beschriebenen monomeren Diisocyanaten ausgewählt ist, dar. Als monomeres Diisocyanat wird hierfür insbesondere ein aromatisches Diisocyanat, insbesondere 2,4- oder 2,6-Toluylendiisocyanat (TDI) oder 4,4'-, 2,4'- oder 2,2'-Diphenylmethandiisocyanat (MDI), bevorzugt.

Für die Umsetzung zum Isocyanatgruppen aufweisenden Polyurethanpolymer **P** geeignete Diole sind insbesondere Polyetherdiole, Polyesterdiole und Polycarbonatdiole, sowie Mischungen dieser Diole.

Als Polyetherdiole, auch Polyoxyalkylendiole genannt, sind insbesondere jene geeignet, welche Polymerisationsprodukte von Ethylenoxid, 1,2-Propylenoxid, 1,2- oder 2,3-Butylenoxid, Tetrahydrofuran oder Mischungen davon sind, eventuell polymerisiert mit Hilfe eines Startermoleküls mit zwei aktiven Wasserstoffatomen wie beispielsweise Wasser, 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, Bisphenol A, hydriertes Bisphenol A, Anilin, sowie kurzkettigere Polyetherdiole, sowie Mischungen der vorgenannten Verbindungen. Eingesetzt werden können sowohl Polyoxyalkylendiole, die einen niedrigen Ungesättigtheitsgrad aufweisen (gemessen nach ASTM D-2849-69 und angegeben in Milliequivalent Ungesättigtheit pro Gramm Diol (mEq/g)), hergestellt beispielsweise mit Hilfe von sogenannten Double Metal Cyanide Complex Katalysatoren (DMC-Katalysatoren), als auch Polyoxyalkylendiole mit einem höheren Ungesättigtheitsgrad, hergestellt beispielsweise mit Hilfe von anionischen Katalysatoren wie NaOH, KOH oder Alkalialkoholaten.

Besonders geeignete Polyoxyalkylendiole sind Polyoxyethylendiole sowie Polyoxypropylendiole.

Besonders geeignet sind Polyoxypropylendiole mit einem Ungesättigtheitsgrad tiefer als 0.02 mEq/g und einem Molekulargewicht im Bereich von 1000 bis 30'000 g/mol, sowie Polyoxypropylendiole mit einem Molekulargewicht von 400 bis 8'000 g/mol. Unter "Molekulargewicht" versteht man im vorliegenden Dokument stets das Molekulargewichtsmittel $M_n$. Insbesondere geeignet sind Polyoxypropylendiole mit einem Ungesättigtheitsgrad tiefer als 0.02 mEq/g und einem Molekulargewicht im Bereich von 1000 bis 12'000 g/mol, insbesondere zwischen 1000 und 8000 g/mol. Solche Polyetherdiole werden beispielsweise unter dem Handelsnamen Acclaim® von Bayer vertrieben.

Ebenfalls besonders geeignet sind sogenannte "EO-endcapped" (ethylene oxide-endcapped) Polyoxypropylendiole. Letztere sind spezielle Polyoxypropylenpolyoxyethylendiole, die beispielsweise dadurch erhalten werden, dass reine Polyoxypropylendiole nach Abschluss der Polypropoxylierung mit Ethylenoxid alkoxyliert werden und dadurch primäre Hydroxylgruppen aufweisen.

Als Polyesterdiole geeignet sind zwei Hydroxylgruppen tragende Polyester, welche nach bekannten Verfahren, insbesondere der Polykondensation von Hydroxycarbonsäuren oder der Polykondensation von aliphatischen und/oder aromatischen Dicarbonsäuren mit zweiwertigen Alkoholen, hergestellt werden.

Insbesondere geeignet sind Polyesterdiole, welche hergestellt sind aus zweiwertigen Alkoholen, wie beispielsweise Ethylenglykol, Diethylenglykol, Propylenglykol, Dipropylenglykol, Neopentylglykol, 1,4-Butandiol, 1,5-Pentandiol, 3-Methyl-1,5-hexandiol, 1,6-Hexandiol, 1,8-Octandiol, 1,10-Decandiol, 1,12-Dodecandiol, 1,12-Hydroxystearylalkohol, 1,4-Cyclohexandimethanol, Dimerfettsäurediol (Dimerdiol), Hydroxypivalinsäureneopentylglykolester oder Mischungen der vorgenannten Alkohole, mit organischen Dicarbonsäuren, oder deren Anhydride oder Ester, wie beispielsweise Bernsteinsäure, Glutarsäure, Adipinsäure, Trimethyladipinsäure, Korksäure, Azelainsäure, Sebacinsäure, Dodecandicarbonsäure, Maleinsäure, Fumarsäure, Dimerfettsäure, Phthalsäure, Phthalsäureanhydrid, Isophthalsäure, Terephthalsäure, Dimethylterephthalat, Hexahydrophthalsäure oder Mischungen der vorgenannten Säuren, sowie Polyesterdiole aus Lactonen wie beispielsweise aus ε-Caprolacton und Startern wie den vorgenannten zweiwertigen Alkoholen.

Insbesondere geeignete Polyesterdiole sind solche, welche aus Adipinsäure, Azelainsäure, Sebacinsäure, Dodecandicarbonsäure, Dimerfettsäure, Phthalsäure, Isophthalsäure und Terephthalsäure als Dicarbonsäure und aus Ethylenglykol, Diethylenglykol, Neopentylglykol, 1,4-Butandiol, 1,6-Hexandiol, Dimerfettsäurediol und 1,4-Cyclohexandimethanol als zweiwertigem Alkohol hergestellt werden. Insbesondere geeignet sind auch Polyesterdiole hergestellt aus ε-Caprolacton und einem der vorgenannten zweiwertigen Alkohole als Starter.

Die Polyesterdiole weisen vorteilhaft ein Molekulargewicht von 1000 bis 15'000 g/mol, insbesondere von 1500 bis 8000 g/mol, insbesondere von 1700 bis 5500 g/mol, auf.

Insbesondere geeignet sind bei Raumtemperatur flüssige, amorphe, teilkristalline und kristalline Polyesterdiole. Besonders geeignet sind bei Raumtemperatur flüssige Polyesterdiole, sowie Mischungen aus amorphen und bei Raumtemperatur flüssigen Polyesterdiolen.

Als Polycarbonatdiole geeignet sind jene, wie sie durch Polykondensation beispielsweise der oben genannten - zum Aufbau der Polyesterdiole eingesetzten - zweiwertigen Alkohole mit Dialkylcarbonaten, wie Dimethylcarbonat, Diarylcarbonaten, wie Diphenylcarbonat, oder Phosgen zugänglich sind.

Besonders geeignet sind bei Raumtemperatur flüssige oder amorphe Polycarbonatdiole.

Für die Umsetzung zum Isocyanatgruppen aufweisenden Polyurethanpolymer **P** weiterhin geeignete Diole sind zwei Hydroxylgruppen tragende Blockcopolymere, welche mindestens zwei verschiedene Blöcke mit Polyether-, Polyester- und/oder Polycarbonatstruktur der oben beschriebenen Art aufweisen.

Für die Umsetzung zum Isocyanatgruppen aufweisenden Polyurethanpolymer **P** bevorzugte Diole sind Polyoxyalkylendiole, insbesondere Polyoxyethylendiole, Polyoxypropylendiole und Polyoxypropylenpolyoxyethylendiole.

Die Herstellung des Isocyanatgruppen aufweisenden Polyurethanpolymers **P** erfolgt in an sich bekannter Art und Weise, entweder direkt aus den Diisocyanaten und den Diolen, oder durch schrittweise Adduktionsverfahren, wie sie auch als Kettenverlängerungsreaktionen bekannt sind.

In einer bevorzugten Ausführungsform wird das Isocyanatgruppen aufweisende Polyurethanpolymer **P** über die Umsetzung von mindestens einem Diisocyanat mit mindestens einem Diol hergestellt, wobei die Isocyanatgruppen gegenüber den Hydroxylgruppen im stöchiometrischen Überschuss vorliegen. Vorteilhaft beträgt das Verhältnis zwischen Isocyanat- und Hydroxylgruppen 1.3 bis 2.5, insbesondere 1.5 bis 2.2.

Das Dialdimin der Formel (I) weist vorzugsweise ein Molekulargewicht von 1'000 bis 30'000 g/mol,insbesondere von 2'000 bis 30'000 g/mol, bevorzugt von 4'000 bis 30'000 g/mol, meist bevorzugt von 6'000 bis 20'000 g/mol, auf.

Das Dialdimin der Formel (I) lässt sich nach dem im Folgenden beschriebenen Verfahren, welches einen weiteren Aspekt der Erfindung darstellt, herstellen.

Dieses Verfahren umfasst den Schritt der Umsetzung eines Diisocyanates **DI** mit einem Dialdimin der Formel (IV), insbesondere der Formel (IV') oder der Formel (IV"), in Gegenwart von Wasser.

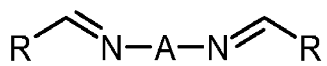

(IV)

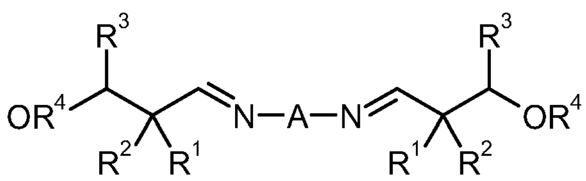

(IV')

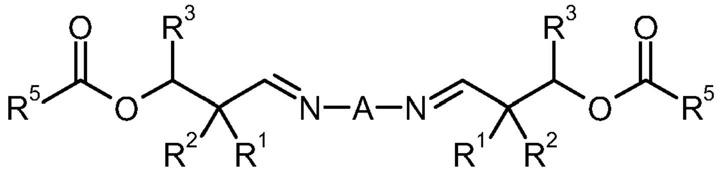

(IV'')

Die Reste R, A, $R^1$, $R^2$, $R^3$ und $R^4$ und $R^5$ sind bereits beschrieben worden.

Diese Umsetzung wird dabei so geführt, dass das molare Verhältnis

$$\frac{[\text{Diisocyanat } \mathbf{DI}]}{[\text{Dialdimin der Formel (IV) oder (IV') oder (IV'')}]} \quad \text{einen Wert von} < 1,$$

insbesondere von 0.9 bis 0.5, bevorzugt von 0.8 bis 0.5, aufweist. Weiterhin ist die Menge des Wassers derart gewählt, dass das molare Verhältnis von [Wasser] / [Diisocyanat **DI**] $\geq 2$, bevorzugt $\geq 10$, ist.

Der gewählte Wert des molaren Verhältnisses von [Diisocyanat **DI**] / [Dialdimin der Formel (IV) oder (IV') oder (IV")] bestimmt dabei direkt die mittlere Kettenlänge, d.h. die Grösse von n in Formel (I) und damit das mittlere Molekulargewicht des Dialdimins der Formel (I). Je näher der Wert des Verhältnisses an 1 liegt, desto grösser ist das Ausmass der Kettenverlängerungsreaktion und desto höher damit das mittlere Molekulargewicht des Dialdimins der Formel (I).

Vorzugsweise wird bei der Herstellung des Dialdimins der Formel (I) zuerst das Diisocyanat **DI** mit dem Dialdimin der Formel (IV) oder (IV') oder (IV") gemischt und anschliessend Wasser hinzugegeben.

Bei dieser Umsetzung wird mindestens ein Aldehyd **ALD** der Formel R-CHO freigesetzt.

Das Dialdimin der Formel (I) wird insbesondere in Form einer Emulsion verwendet.

Ein weiterer Aspekt der vorliegenden Erfindung betrifft somit eine aldiminhaltige Emulsion.

Die aldiminhaltige Emulsion umfasst

a) mindestens ein Dialdimin der Formel (I), wie es vorgängig beschrieben wurde;
b) Wasser; und
c) gegebenenfalls mindestens ein Tensid.

Es ist auch möglich, dass die aldiminhaltige Emulsion aus a), b) und gegebenenfalls c) besteht. Vorzugsweise umfasst eine aldiminhaltige Emulsion mindestens ein Tensid.

Als Tenside können natürliche oder synthetische Stoffe verwendet werden, welche in Lösungen die Oberflächenspannung des Wassers oder anderer Flüssigkeiten herabsetzen. Als Tenside, auch Netzmittel genannt, können anionische, kationische, nichtionische oder ampholytische Tenside oder deren Mischungen verwendet werden.

Beispiele für anionische Tenside sind Carboxylat-, Sulfat-, Phosphat- oder Sulfonat-Gruppen aufweisende Verbindungen, wie zum Beispiel Fettalkoholethersulfate, Fettalkoholsulfate, Seifen, Alkylsulfonate, Olefinsulfonate, Arylsulfonate, Alkylarylsulfonate und Alkylphosphate.

Beispiele für kationische Tenside sind quaternäre Ammonium- oder Phosphoniumverbindungen, wie zum Beispiel Tetraalkylammoniumsalze, Dimethyldistearyl-ammoniumsalze und N-Alkylpyridiniumsalze, insbesondere die Chloride davon.

Zu den nichtionischen Tensiden, den sogenannten Niotensiden, gehören beispielsweise Ethoxylate von Alkoholen, Phenolen, Alkylphenolen, Aminen, Fettaminen, Fettsäuren, Fettsäureamiden, Polysiloxanen und Fettsäureester, auch Alkyl- oder Alkylphenyl-polyglykolether, wie zum Beispiel Fettalkoholpolyglykolether, oder Fettsäureamide, Alkylglykoside, Zuckerester, Sorbitanester, Polysorbate oder Trialkylaminoxide, auch Ester und Amide von Poly(meth) acrylsäuren mit Polyalkylenglykolen oder Aminopolyalkylenglykolen, die einseitig mit Alkylgruppen abgeschlossen sein können.

Zu den ampholytischen oder amphoterischen Tensiden gehören amphotere Elektrolyte, sogenannte Ampholyte, wie zum Beispiel Aminocarbonsäuren, und Betaine.

Derartige Tenside sind kommerziell breit erhältlich.

Als besonders geeignet haben sich anionische Tenside gezeigt.

Vorteilhaft wird die aldiminhaltige Emulsion dadurch erhalten, dass dem Dialdimin der Formel (I) Wasser sowie gegebenenfalls das Tensid unter Rühren beigegeben werden.

Es ist dem Fachmann klar, dass die aldiminhaltige Emulsion auch anders hergestellt werden kann.

Zwar ist dem Fachmann bekannt, dass bei der Herstellung einer Emulsion verschiedene Parameter, wie Temperatur, Rührgeschwindigkeit und Art und Geometrie der Rührwerkzeuge, einen wichtigen Einfluss auf die Qualität der Emulsion ausüben. Es zeigte sich jedoch, dass diese Parameter bei der Herstellung der hier besprochenen aldiminhaltigen Emulsion eher unkritisch sind. So liess sich die aldiminhaltige Emulsion auf verschiedensten Rührwerken ohne grosse Anpassung der Rührparameter schnell, reproduzierbar und in guter Qualität herstellen.

Es kann weiterhin von Vorteil sein, dass die aldiminhaltige Emulsion weitere Bestandteile umfasst. Insbesondere als weitere Bestandteile zu nennen sind Co-Emulgatoren, Entschäumungsmittel, Stabilisatoren, Biozide, wie Fungizide, Bakteriozide oder Algizide, Haftvermittler, Lösemittel, Weichmacher, Katalysatoren, Füllstoffe und Rheologiehilfsmittel, insbesondere Verdickungsmittel und Thixotropiermittel.

Die aldiminhaltige Emulsion kann organische Lösemittel aufweisen oder nicht. Wenn organische Lösemittel eingesetzt werden, ist es vorteilhaft, wenn diese keine VOC-Lösemittel (VOC = Volatile Organic Compounds) sind. Es gibt jedoch sehr viele unterschiedliche Definitionen von "flüchtigen organischen Verbindungen" bzw. "VOC". So wird beispielsweise ein VOC nach der EU-Richtline 2004/42/EG als eine organische Verbindung mit einem Siedepunkt von höchstens 250 °C bei einem Standarddruck von 101,3 kPa definiert. Gemäss der Schweizer Verordnung über die Lenkungsabgabe auf flüchtige organische Verbindungen werden VOC als organische Verbindungen mit einem Dampfdruck von mindestens 0.1 mbar bei 20 °C oder einem Siedepunkt von höchstens 240 °C bei 1013.25 mbar definiert. Im vorliegenden Dokument gelten als "flüchtige organische Verbindungen" oder "VOC" organische Verbindungen, welche einen Siedepunkt von höchstens 250 °C bei Normdruck (1013 mbar) oder einen Dampfdruck von mindestens 0.1 mbar bei 20 °C aufweisen.

Insbesondere handelt es sich bei derartigen Lösemitteln, welche nicht VOC sind, um Carbonsäureester.

Die aldiminhaltige Emulsion ist bevorzugt frei von N-Alkylpyrrolidonen. Insbesondere enthält die aldiminhaltige

Emulsion kein N-Methylpyrrolidon (NMP).

Als Katalysatoren eignen sich vor allem tertiäre Amine, insbesondere Trialkylamine wie beispielsweise Triethylamin, sowie Morpholinether-Derivate wie Dimorpholinodiethylether, Tetramethyl-dimorpholinodiethylether, Polyethylenglycoldimorphonlinoether und dergleichen.

Es hat sich als besonders vorteilhaft erwiesen, wenn die aldiminhaltige Emulsion neben mindestens einem Dialdimin der Formel (I) und Wasser zusätzlich mindestens ein Tensid, mindestens einen Füllstoff, insbesondere eine Kreide oder eine pyrogene Kieselsäure aufweist. Weiterhin weist die aldiminhaltige Emulsion vorzugsweise mindestens einen Katalysator auf.

Vorzugsweise hat die aldiminhaltige Emulsion eine pastöse Konsistenz, d.h. sie ist nicht selbstfliessend.

Die aldiminhaltige Emulsion ist stabil und kann verpackt und gelagert werden. Bei der Lagerung der aldiminhaltigen Emulsion ist jedoch darauf zu achten, dass die Temperatur nicht unter den Gefrierpunkt der Emulsion fällt, da sonst die Gefahr besteht, dass die Emulsion bricht. Eine gebrochene Emulsion kann bei ihrer Verwendung schwerwiegende Nachteile zur Folge haben.

Die beschriebene aldiminhaltige Emulsion ist breit anwendbar. In einer bevorzugten Ausführungsform wird die aldiminhaltige Emulsion als Härterkomponente oder als Beschleunigerkomponente für einen Klebstoff, Dichtstoff oder Beschichtungsstoff, welcher Isocyanatgruppen aufweisende Polymere, insbesondere Isocyanatgruppen aufweisende Polyurethanpolymere, enthält, verwendet.

In einem weiteren Aspekt betrifft die vorliegende Erfindung somit eine zweikomponentige Zusammensetzung, welche aus den zwei Komponenten **K1** und **K2** besteht.

Die erste Komponente, Komponente **K1,** enthält mindestens ein Isocyanatgruppen aufweisendes Polymer, insbesondere mindestens ein Isocyanatgruppen aufweisendes Polyurethanpolymer **P,** wie vorgängig beschrieben, oder besteht daraus.

Die zweite Komponente, Komponente **K2**, enthält ein Dialdimin der Formel (I), wie vorgängig beschrieben, oder eine aldiminhaltige Emulsion, wie vorgängig beschrieben, oder besteht daraus.

Vorteilhaft enthält die Komponente **K1** mindestens einen Füllstoff. Der Füllstoff beeinflusst beispielsweise sowohl die Konsistenz der nicht ausgehärteten Zusammensetzung als auch die mechanischen Eigenschaften der ausgehärteten Zusammensetzung. Geeignete Füllstoffe sind anorganische und organische Füllstoffe, zum Beispiel natürliche, gemahlene oder gefällte Calciumcarbonate, welche gegebenenfalls mit Fettsäuren, insbesondere Stearaten, beschichtet sind, calcinierte Kaoline, Aluminiumoxide, Aluminiumhydroxide, Baryt ($BaSO_4$, auch Schwerspat genannt), Kieselsäuren, insbesondere hochdisperse Kieselsäuren aus Pyrolyseprozessen, Russe, insbesondere industriell hergestellte Russe (im Folgenden als "Russ" bezeichnet), PVC-Pulver oder Hohlkugeln. Bevorzugte Füllstoffe sind Calciumcarbonate, Kaoline, pyrogene Kieselsäuren und Russ. Es kann von Vorteil sein, eine Mischung verschiedener Füllstoffe einzusetzen.

Eine geeignete Menge Füllstoff liegt beispielsweise im Bereich von 10 bis 70 Gewichts-%, bevorzugt 20 bis 60 Gewichts-%, bezogen auf die gesamte Komponente **K1.**

Vorteilhaft enthält die Komponente **K1** weiterhin mindestens einen Katalysator, welcher die Reaktion der Isocyanatgruppen und / oder die Hydrolyse der Aldimingruppen beschleunigt.

Katalysatoren, welche die Reaktion der Isocyanatgruppen mit Wasser beschleunigen, sind insbesondere Metallverbindungen, beispielsweise Zinnverbindungen wie Dibutylzinndiacetat, Dibutylzinndilaurat, Dibutylzinndistearat, Dibutylzinndiacetylacetonat, Dioctylzinndilaurat, Dibutylzinndichlorid und Dibutylzinnoxid, Zinn(II)-carboxylate, Stannoxane wie Laurylstannoxan, Bismutverbindungen wie Bismut(III)-octoat, Bismut(III)-neodecanoat oder Bismut (III)-oxinate; sowie tertiäre Amine, beispielsweise 2,2'-Dimorpholinodiethylether und andere Morpholinether-Derivate.

Katalysatoren, welche die Hydrolyse von Aldimingruppen beschleunigen, sind insbesondere Säuren oder zu Säuren hydrolysierbare Verbindungen, beispielsweise organische Carbonsäuren wie Benzoesäure, Salicylsäure oder 2-Nitrobenzoesäure, organische Carbonsäureanhydride wie Phthalsäureanhydrid oder Hexahydrophthalsäureanhydrid, Silylester von organischen Carbonsäuren, organische Sulfonsäuren wie Methansulfonsäure, p-Toluolsulfonsäure oder 4-Dodecylbenzolsulfonsäure, oder weitere organische oder anorganische Säuren.

Es können auch Kombinationen der genannten Katalysatoren vorhanden sein, insbesondere Mischungen von Säuren und Metallverbindungen, oder von Metallverbindungen und tertiären Aminen, oder von Säuren und tertiären Aminen, oder von Säuren und Metallverbindungen und tertiären Aminen.

Ein typischer Gehalt an Katalysator beträgt 0.005 bis 1 Gewichts-%, bezogen auf die gesamte Komponente **K1,** wobei dem Fachmann klar ist, welche Einsatzmengen für welche Katalysatoren hierfür sinnvoll sind.

Vorteilhaft enthält die Komponente **K1** weiterhin mindestens einen Weichmacher. Geeignete Weichmacher sind beispielsweise Ester organischer Carbonsäuren oder deren Anhydride, zum Beispiel Phthalate wie Dioctylphthalat, Diisononylphtalat oder Diisodecylphthalat, Adipate wie Dioctyladipat, Azelate und Sebacate; organische Phosphor- und Sulfonsäureester und Polybutene.

Weitere mögliche Bestandteile der Komponente **K1** sind unter anderem die folgenden Hilfs- und Zusatzmittel:

- Lösemittel, insbesondere Lösemittel, welche nicht VOC sind;
- Fasern, beispielsweise aus Polyethylen;
- Pigmente, beispielsweise Titandioxid oder Eisenoxide;
- weitere in der Polyurethanchemie übliche Katalysatoren;
- Rheologie-Modifizierer wie beispielsweise Verdickungsmittel oder Thixotropiermittel, zum Beispiel Harnstoffverbindungen, Polyamidwachse, Bentonite oder pyrogene Kieselsäuren;
- Reaktivverdünner oder Vernetzer, beispielsweise Oligomere oder Polymere von Diisocyanaten wie MDI, PMDI, TDI, HDI, 1,12-Dodecamethylendiisocyanat, Cyclohexan-1,3- oder 1,4-diisocyanat, IPDI, Perhydro-2,4'- und - 4,4'-diphenylmethandiisocyanat ($H_{12}$MDI), 1,3- und 1,4-Tetramethylxylylendiisocyanat, insbesondere Isocyanurate, Carbodiimide, Uretonimine, Biurete, Allophanate und Iminooxadiazindione der genannten Diisocyanate, Addukte von Diisocyanaten mit kurzkettigen Polyolen, Adipinsäuredihydrazid und andere Dihydrazide, sowie blockierte Härter in Form von Polyaldiminen, Polyketiminen, Oxazolidinen oder Polyoxazolidinen;
- Trocknungsmittel, wie beispielsweise Molekularsiebe, Calciumoxid, hochreaktive Isocyanate wie p-Tosylisocyanat, Orthoameisensäureester, Alkoxysilane wie Tetraethoxysilan, Organoalkoxysilane wie Vinyltrimethoxysilan, und Organoalkoxysilane, welche in $\alpha$-Stellung zur Silangruppe eine funktionelle Gruppe aufweisen;
- Haftvermittler, insbesondere Organoalkoxysilane, im Folgenden "Silane" genannt, wie beispielsweise Epoxysilane, Vinylsilane, (Meth)acrylsilane, Isocyanatosilane, Carbamatosilane, S-(Alkylcarbonyl)-mercaptosilane und Aldiminosilane, sowie oligomere Formen dieser Silane;
- Stabilisatoren gegen Wärme, Licht- und UV-Strahlung;
- flammhemmende Substanzen;
- oberflächenaktive Substanzen wie beispielsweise Netzmittel, Verlaufsmittel, Entlüftungsmittel oder Entschäumer;
- Biozide, wie beispielsweise Algizide, Fungizide oder das Pilzwachstum hemmende Substanzen;

sowie weitere, üblicherweise in einkomponentigen Polyurethanzusammensetzungen eingesetzte Substanzen.

Besonders deutlich sind die Vorteile der vorliegenden Erfindung in den Fällen ersichtlich, in denen die Komponente **K1** als weitere Bestandteile sowohl Füllstoffe als auch Polyaldimine enthält.

Es ist vorteilhaft, alle der genannten, in der Komponente **K1** gegebenenfalls enthaltenen Bestandteile, insbesondere einen Füllstoff, einen Katalysator sowie einen Weichmacher, so auszuwählen, dass die Lagerstabilität der Komponente **K1** durch die Anwesenheit eines solchen Bestandteils nicht beeinträchtigt ist, das heisst, dass sich die Komponente **K1** in ihren Eigenschaften, insbesondere den Applikations- und Aushärtungseigenschaften, bei der Lagerung nicht oder nur wenig verändert. Dies bedingt, dass zur chemischen Aushärtung der Komponente **K1** führende Reaktionen, insbesondere der Isocyanatgruppen, während der Lagerung nicht in signifikantem Ausmass auftreten. Es ist deshalb insbesondere wichtig, dass die genannten Bestandteile kein oder höchstens Spuren von Wasser enthalten oder beim Lagern freisetzen. Es kann sinnvoll sein, gewisse Bestandteile vor dem Einmischen in die Komponente **K1** chemisch oder physikalisch zu trocknen.

Die Komponente **K1** wird unter Ausschluss von Feuchtigkeit hergestellt und aufbewahrt. In einer geeigneten, klimadichten Verpackung oder Anordnung, wie beispielsweise in einem Fass, einem Beutel oder einer Kartusche, verfügt sie über eine hervorragende Lagerstabilität. Mit den Begriffen "lagerstabil" und "Lagerstabilität" in Zusammenhang mit einer Zusammensetzung wird im vorliegenden Dokument der Sachverhalt bezeichnet, dass die Viskosität der Zusammensetzung bei gegebener Applikationstemperatur und bei geeigneter Lagerung in der betrachteten Zeitspanne nicht oder höchstens so stark ansteigt, dass die Zusammensetzung auf die vorgesehene Weise verwendbar bleibt.

Typischerweise ist die Komponente **K1** ein feuchtigkeitshärtender einkomponentiger Polyurethan-Klebstoff oder -Dichtstoff, wie sie kommerziell breit erhältlich sind. Beispielsweise werden derartige Klebstoffe unter dem Namen Sikaflex® oder SikaTack® von Sika Schweiz AG angeboten.

Die Komponente **K1** reagiert für sich allein genommen, das heisst ohne Komponente **K2**, mit Luftfeuchtigkeit und härtet dadurch aus. Allerdings ist diese Aushärtung sehr langsam; ausserdem tritt bei einer derartigen Aushärtung gelegentlich Blasenbildung auf. Durch die beschriebene Verwendung der zweiten (Aldimin-enthaltenden) Komponente **K2** wird jedoch die Aushärtung stark beschleunigt und die Blasenbildung unterdrückt.

Die Komponente **K2** kann weitere Bestandteile enthalten, insbesondere solche, wie sie für die Komponente **K1** oben aufgeführt sind, unter der Bedingung, dass diese weiteren Bestandteile mit den anderen Bestandteilen der Komponente **K2** lagerstabil sind. Die Komponente **K2** wird nach der Herstellung in eine geeignete, verschlossene Verpackung oder Anordnung, wie beispielsweise in ein Fass, einen Beutel oder eine Kartusche, abgefüllt und kann in dieser Verpackung gelagert und transportiert werden.

Es ist auch möglich, dass die Verpackungen der Komponenten **K1** und **K2** miteinander verbunden sind. So ist es beispielsweise möglich, Komponenten **K1** und **K2** in je eine Kammer einer Doppelkartusche abzufüllen. Beispielsweise sind dies sogenannte Coaxialkartuschen oder Zwillingskartuschen. Derartige Verpackungen, welche in örtlich voneinander getrennten Kammern Komponente **K1** und **K2** enthalten, sind insofern vorteilhaft, als die zwei Komponenten bei der Lagerung und beim Transport untrennbar oder unverlierbar zusammen bleiben und somit für die Applikation der

zweikomponentigen Zusammensetzung nicht zuerst zusammengesucht, bzw. zusammengestellt, werden müssen.

Die zwei Komponenten **K1** und **K2** werden vor oder während der Applikation der zweikomponentigen Zusammensetzung miteinander vermischt. Die Vermischung kann auf verschiedene Art und Weise erfolgen.

Insbesondere kann die Vermischung weitgehend homogen oder inhomogen erfolgen. Die Vermischung der zwei Komponenten erfolgt vorzugsweise über einen Statikmischer oder über einen dynamischen Mischer. Daraus resultiert eine vermischte oder teilvermischte zweikomponentige Zusammensetzung.

Die Figuren 1a bis 1f zeigen einige Beispiele für derartige Vermischungen. Die Figuren 2a bis 2f zeigen Beispiele für Mischvorrichtungen, mit welchem derartige Mischbilder erreicht werden können.

In einer Ausführungsform erfolgt die Vermischung der beiden Komponenten **K1** und **K2** im Wesentlichen homogen.

Dies kann insbesondere durch die Verwendung von dynamischen Mischern erreicht werden. Es ist auch möglich, durch die Verwendung von Statikmischern mit vielen Mischelementen zu einer im Wesentlichen homogenen Vermischung zu kommen.

Wie der Fachmann weiss, ist der Term "homogen vermischt" im Kontext von pastösen Kleb- und Dichtstoffen, wie sie hier besprochen sind, nicht absolut zu verstehen. Der Term bedeutet in der üblichen Verwendung nämlich, dass von Auge keine Mischgrenzen mehr sichtbar sind, während dies beispielsweise unter dem Mikroskop durchaus noch möglich ist. Aufgrund von experimentellen Erkenntnissen kann davon ausgegangen werden, dass bei der Verwendung von Statikmischern des Typs Sulzer Quadro® (erhältlich von der Firma Sulzer Chemtech) mit 18 oder mehr Statikmischer-Elementen die Vermischung von zwei pastösen Komponenten im Wesentlichen homogen erfolgt.

In einer weiteren Ausführungsform erfolgt die Vermischung der beiden Komponenten **K1** und **K2** im Wesentlichen inhomogen, beispielsweise in Form einer schichtenartigen oder einer strangartigen Vermischung.

Eine schichtenartige Vermischung kann insbesondere durch die Verwendung von Statikmischern erreicht werden, wobei diese eine begrenzte Zahl von Statikmischer-Elementen aufweisen. Hierfür kann beispielsweise ein Dosieradapter, wie er in WO 95/24556 A1 beschrieben ist, deren gesamte Inhalt hiermit in die Offenbarung der vorliegenden Erfindung mit eingeschlossen wird, und mittels welchem die Komponente **K2** der Komponente **K1** zugemischt wird, eingesetzt werden.

Die Zahl der Statikmischer-Elemente beträgt insbesondere 6 bis 16, bevorzugt 6 bis 12.

Eine strangartige Vermischung ergibt sich in einer Variante dadurch, dass ein oder mehrere Stränge der Komponente **K2** von der Komponente **K1** ummantelt werden. Dies kann durch eine Applikationsapparatur, wie sie in EP 1 728 840 A1 beschrieben ist, erfolgen. Der gesamte Inhalt dieses Dokumentes wird hiermit in die Offenbarung der vorliegenden Erfindung mit eingeschlossen. In einer weiteren Variante wird ein Strang aus den miteinander vermischten Komponenten **K1** und **K2** von der Komponente **K1** ummantelt. Diese Variante hat den Vorteil, dass im Kern der Raupe, das heisst dort, wo die Komponenten **K1** und **K2** vermischt sind, eine schnelle Aushärtung erfolgt, während die Oberfläche der Raupe im Wesentlichen lediglich über Luftfeuchtigkeit aushärtet, da dort keine Komponente **K2** vorhanden ist. Dies führt dazu, dass die Offenzeit (beschränkt durch die Hautbildung) einer derartigen Raupe im Gegensatz zu einer Raupe, welche über den gesamten Querschnitt aus den vermischten Komponenten **K1** und **K2** besteht, verlängert ist, so dass die Vorteile einer langer Offenzeit und einer schnellen Aushärtung kombiniert werden. Eine Möglichkeit, eine derartige Ummantelung zu erzielen, bietet ein Statikmischer, wie er in WO 02/32562 A1 offenbart wird, und deren gesamte Inhalt hiermit in die Offenbarung der vorliegenden Erfindung mit eingeschlossen wird.

Es hat sich besonders bei feuchtigkeitshärtenden Polyurethan-Klebstoffen mit hohem Elastizitätsmodul als Komponente **K1 -** vor allem bei solchen, welche Polyaldimine enthalten - gezeigt, dass die nach der inhomogenen Zumischung der Komponente **K2** erhaltene Zusammensetzung eine ausserordentlich gute Verarbeitbarkeit sowie eine hohe Endfestigkeit aufweist. Dies steht in Gegensatz zu Zusammensetzungen, wie sie aus der analogen Verwendung von Komponenten **K2** nach dem Stand der Technik, vor allem N-Alkylpyrrolidon enthaltende Komponenten **K2,** entstehen. In diesen Fällen ist die Verarbeitbarkeit oft schon kurze Zeit nach dem Zumischen der Komponente **K2** ungenügend, was sich beispielsweise dadurch äussert, dass ein an der Zusammensetzung vorgenommener Abstrich einen griesigen Aspekt aufweist. Weiterhin bleibt die Endfestigkeit der ausgehärteten Zusammensetzung beschränkt. Dies ist möglicherweise auf eine ungenügende Einbindung der in der Komponente **K2** enthaltenen Bestandteile in die Polyurethanmatrix zurückzuführen, was dazu führt, dass die ausgehärtete Zusammensetzung bei Kraftbelastung innerhalb der ehemaligen Schichten der Komponente **K2** reisst.

Die beschriebene zweikomponentige Zusammensetzung kann als Klebstoff, Dichtstoff oder Beschichtung oder Belag verwendet werden.

In der Anwendung als Klebstoff wird die zweikomponentige Zusammensetzung zum Verkleben eines Substrates **S1** und eines Substrates S2 eingesetzt. Das Verkleben erfolgt vorzugsweise nach dem im Folgenden beschriebenen Verfahren.

Dieses Verfahren des Verklebens von Substraten **S1** und **S2** umfasst die Schritte

i) Applizieren einer vorgängig beschriebenen zweikomponentigen Zusammensetzung während des Mischens oder

nach dem Mischen der zwei Komponenten **K1** und **K2** auf ein Substrat **S1;**
ii) Kontaktieren der applizierten Zusammensetzung mit einem Substrat **S2;**
oder

i') Applizieren einer vorgängig beschriebenen zweikomponentigen Zusammensetzung während des Mischens oder nach dem Mischen der zwei Komponenten **K1** und **K2** auf ein Substrat **S1;**
ii') Applizieren einer vorgängig beschriebenen zweikomponentigen Zusammensetzung während des Mischens oder nach dem Mischen der zwei Komponenten **K1** und **K2** auf ein Substrat **S2;**
iii') Kontaktieren der auf Substrat **S1** applizierten Zusammensetzung mit der auf Substrat **S2** applizierten Zusammensetzung.

Die Substrate **S1** und **S2** sind hierbei gleich oder verschieden voneinander.
In der Anwendung als Dichtstoff wird die zweikomponentige Zusammensetzung zum Abdichten von Substraten eingesetzt. Das Abdichten erfolgt vorzugsweise nach dem im Folgenden beschriebenen Verfahren.
Dieses Verfahren des Abdichtens umfasst den Schritt

i") Applizieren einer vorgängig beschriebenen zweikomponentigen Zusammensetzung während des Mischens oder nach dem Mischen der zwei Komponenten **K1** und **K2** zwischen ein Substrat **S1** und ein Substrat **S2.**

Die Substrate **S1** und **S2** sind hierbei gleich oder verschieden voneinander.
Bei der Anwendung als Dichtstoff wird die Zusammensetzung als Abdichtung zwischen zwei Substraten **S1** und **S2** eingesetzt. Üblicherweise wird der Dichtstoff in eine Fuge eingepresst.
Die Herstellung einer Beschichtung erfolgt vorzugsweise nach dem im Folgenden beschriebenen Verfahren.
Dieses Verfahren zur Herstellung einer Beschichtung umfasst den Schritt

i''') Applizieren einer vorgängig beschriebenen zweikomponentigen Zusammensetzung während des Mischens oder nach dem Mischen der zwei Komponenten **K1** und **K2** auf ein Substrat **S1.**

In diesen drei Verfahren erfolgt das Mischen der zwei Komponenten **K1** und **K2** in einer Ausführungsform im Wesentlichen homogen. In einer anderen Ausführungsform erfolgt das Mischen der zwei Komponenten **K1** und **K2** im Wesentlichen inhomogen. Insbesondere erfolgt die inhomogene Vermischung derart, dass ein schichtenartiges, strangförmiges oder spiralförmiges Mischbild gebildet wird.
Gegebenenfalls kann sich anschliessend an Schritt ii), iii'), i") oder i''') ein Schritt des Aushärtens mittels Luftfeuchtigkeit anschliessen.
Geeignete Substrate **S1** oder **S2** sind beispielsweise anorganische Substrate wie Glas, Glaskeramik, Beton, Mörtel, Backstein, Ziegel, Gips und Natursteine wie Granit oder Marmor; Metalle oder Legierungen wie Aluminium, Stahl, Buntmetalle, verzinkte Metalle; organische Substrate wie Holz, Kunststoffe wie PVC, Polycarbonate, PMMA, Polyester, Epoxidharze; beschichtete Substrate wie pulverbeschichtete Metalle oder Legierungen; sowie Farben und Lacke. Bevorzugt ist das Substrat **S1** und/oder **S2** ausgewählt aus der Gruppe bestehend aus Beton, Zement, Mörtel, Backstein, Ziegel, Gips, Naturstein, Asphalt, Metall, Metalllegierung, Holz, Keramik, Glas, Kunststoff, Pulverbeschichtung, Farbe und Lack.
Die Substrate können bei Bedarf vor dem Applizieren der zweikomponentigen Zusammensetzung vorbehandelt werden. Derartige Vorbehandlungen umfassen insbesondere physikalische und/oder chemische Reinigungsverfahren, beispielsweise Schleifen, Sandstrahlen, Bürsten oder dergleichen, oder Behandeln mit Reinigern oder Lösemitteln oder das Aufbringen eines Haftvermittlers, einer Haftvermittlerlösung oder eines Primers.
Besonders vorteilhaft wird das Verfahren zum Einglasen von Scheiben in Transportmitteln, insbesondere in Automobile verwendet.
Aus einem der beschriebenen Verfahren der Verklebung, bzw. der Abdichtung, bzw. zur Herstellung einer Beschichtung, resultiert ein Artikel.
Der Artikel kann ein Gebäude oder ein Bauwerk des Hoch- oder Tiefbaus, ein industriell gefertigtes Gut oder ein Konsumgut, insbesondere ein Fenster, eine Haushaltsmaschine, oder ein Transportmittel, insbesondere ein Fahrzeug, oder ein Anbauteil eines Fahrzeugs, sein.
Die beschriebene zweikomponentige Zusammensetzung weist die grossen Vorteile auf, einerseits eine ausgezeichnete Frühfestigkeit und eine hohe Endfestigkeit aufzuweisen und andererseits frei von VOC-Lösemitteln, insbesondere frei von N-Alkylpyrrolidonen, formulierbar zu sein und damit bezüglich ökologischer und arbeitshygienischer Eigenschaften hohen Standards zu genügen.

## Kurze Beschreibung der Zeichnung

Im Folgenden werden anhand der Zeichnungen ausgewählte Ausführungsbeispiele der Erfindung näher erläutert. Gleiche Elemente sind in den verschiedenen Figuren mit den gleichen Bezugszeichen versehen. Die Richtung von Kräften, beziehungsweise Bewegungen, ist mit Pfeilen angegeben.
Es zeigen:

Fig. 1a-d eine schematische Querschnitt-Darstellung durch eine Dreiecksraupe 1 quer zur Raupenrichtung
- Fig. 1a strangförmiges Mischbild mit einem Strang **K2**
- Fig. 1b strangförmiges Mischbild mit mehreren Strängen **K2**
- Fig. 1c strangförmiges Mischbild eines mit **K1** umhüllten Strang aus **K1/K2.**
- Fig. 1d schichtenartiges Mischbild

Fig. 1e-f eine schematische Querschnitt-Darstellung durch eine Dreiecksraupe 1 in Raupenrichtung
- Fig. 1e strangförmiges Mischbild mit einem Strang **K2**
- Fig. 1b spiralförmiges Mischbild mit einem Strang **K2**

Fig. 2a-f eine schematische Querschnitt-Darstellung entlang der Längsachse z durch eine Mischvorrichtung
- Fig. 2a Mischvorrichtung führend zu einem Mischbild nach Fig. 1a)
- Fig. 2b Mischvorrichtung führend zu einem Mischbild nach Fig. 1b)
- Fig. 2c Mischvorrichtung führend zu einem Mischbild nach Fig. 1c)
- Fig. 2d Mischvorrichtung führend zu einem Mischbild nach Fig. 1d)
- Fig. 2f Mischvorrichtung führend zu einem Mischbild nach Fig. 1f)
- Fig. 2f' Detail zum Endstück 14

Es sind nur die für das unmittelbare Verständnis der Erfindung wesentlichen Elemente gezeigt. Nicht dargestellt sind beispielsweise die Vorratsbehälter und Details zur Zuführung der Komponenten **K1** und **K2.**
Figur 1a ist eine schematische Darstellung eines Querschnitts durch eine Dreiecksraupe 1 quer zur Raupenrichtung, nach einer teilweisen Vermischung von Komponenten **K1** und **K2** einer zweikomponentigen Zusammensetzung, welche auf ein Substrat **S1** appliziert wurde. In diesem Beispiel erfolgt die teilweise Vermischung so, dass ein strangförmiges Mischbild entsteht, bei welchem ein Strang der Komponente **K2** von Komponente **K1** umhüllt ist.
Ein derartiges Mischbild lässt sich beispielsweise durch eine Mischvorrichtung, wie sie schematisch in Figur 2a dargestellt ist, erreichen.
Figur 1b ist eine schematische Darstellung eines Querschnitts durch eine Dreiecksraupe 1 quer zur Raupenrichtung, nach einer teilweisen Vermischung von Komponenten **K1** und **K2** einer zweikomponentigen Zusammensetzung, welche auf ein Substrat **S1** appliziert wurde. In diesem Beispiel erfolgt die teilweise Vermischung so, dass ein strangförmiges Mischbild entsteht, bei welchem mehrere Stränge der Komponente **K2** von **K1** umhüllt sind.
Ein derartiges Mischbild lässt sich beispielsweise durch eine Mischvorrichtung, wie sie schematisch in Figur 2b dargestellt ist, erreichen.
Figur 1c ist eine schematische Darstellung eines Querschnitts durch eine Dreiecksraupe 1 quer zur Raupenrichtung, nach einer teilweisen Vermischung von Komponenten **K1** und **K2** einer zweikomponentigen Zusammensetzung, welche auf ein Substrat **S1** appliziert wurde. In diesem Beispiel erfolgt die teilweise Vermischung, indem ein Mischbereich 2, wo die Komponenten **K1** und **K2** vollständig oder teilweise miteinander vermischt sind, von der unvermischten Komponente **K1** umhüllt ist. In der hier dargestellten Ausführungsform ist die Vermischung im Mischbereich 2 in einer schichtenartig.
Ein derartiges Mischbild lässt sich beispielsweise durch eine Mischvorrichtung, wie sie schematisch in Figur 2c dargestellt ist, erreichen.
Figur 1d ist eine schematische Darstellung eines Querschnitts durch eine Dreiecksraupe 1 quer zur Raupenrichtung, nach einer teilweisen Vermischung von Komponenten **K1** und **K2** einer zweikomponentigen Zusammensetzung, welche auf ein Substrat **S1** appliziert wurde. In diesem Beispiel erfolgt die teilweise Vermischung schichtenartig.
Ein derartiges Mischbild lässt sich beispielsweise durch eine Mischvorrichtung, wie sie schematisch in Figur 2d dargestellt ist, erreichen.
Figur 1e ist eine schematische Darstellung eines Querschnitts durch eine Dreiecksraupe 1 in Raupenrichtung, nach einer teilweisen Vermischung von Komponenten **K1** und **K2** einer zweikomponentigen Zusammensetzung, welche auf ein Substrat **S1** appliziert wurde. In diesem Beispiel erfolgt die teilweise Vermischung in der Form eines Stranges **K2,** welcher von **K1** umhüllt ist, und welcher co-linear zur Raupe orientiert ist, so dass ein strangförmiges Mischbild entsteht.
Ein derartiges Mischbild lässt sich beispielsweise durch eine Mischvorrichtung, wie sie schematisch in Figur 2a dargestellt ist, erreichen.
Figur 1f ist eine schematische Darstellung eines Querschnitts durch eine Dreiecksraupe 1 in Raupenrichtung,

nach einer teilweisen Vermischung von Komponenten **K1** und **K2** einer zweikomponentigen Zusammensetzung, welche auf ein Substrat **S1** appliziert wurde. In diesem Beispiel erfolgt die teilweise Vermischung in der Form eines Stranges **K2,** welcher von **K1** umhüllt ist, und welcher in der Klebstoffraupe spiralförmig orientiert ist, so dass ein spiralförmiges Mischbild entsteht.

Ein derartiges Mischbild lässt sich beispielsweise durch eine Mischvorrichtung, wie sie schematisch in Figur 2f dargestellt ist, erreichen.

Figur 2a zeigt schematisch einen Querschnitt entlang der Längsachse z durch eine Mischvorrichtung 3, welche zu einem strangförmigen Mischbild, wie in Figur 1a gezeigt, führt. Die Mischvorrichtung 3 weist eine Zuführungsleitung 4 für die Komponente **K1,** auf, die rohrförmig einen Innenraum 9 begrenzt, in welchem die Komponente **K1** zugeführt wird. In diesen Innenraum 9 ragt eine Zuführungsleitung 6 für die Komponente **K2** hinein. Bei der Applikation, bzw. der Vermischung, werden die Komponente **K1** durch die Zuführungsleitung 4 und die Komponente **K2** durch die Zuführungsleitung 6 gepresst. Nach dem Passieren der Austrittsöffnung 8 der Zuführungsleitung für die Komponente **K2** sind die zwei Komponenten **K1** und **K2** in Flussrichtung abwärts miteinander in Kontakt, und es entsteht eine teilweise Vermischung der zwei Komponenten. Beim Austritt aus der Austrittsöffnung 7 der Mischvorrichtung zeigt sich ein strangförmiges Mischbild, bei welchem die Komponente **K2** von der Komponente **K1** umhüllt ist.

Die Figur 2b zeigt schematisch einen Querschnitt entlang der Längsachse z durch eine Mischvorrichtung 3', welche zu einem strangförmigen Mischbild, wie in Figur 1b gezeigt, führt. Die Mischvorrichtung 3' ist eine Variante der Mischvorrichtung 3, die vorgängig in Figur 2a diskutiert wurde. Bei dieser Variante weist die in den Innenraum 9 ragende Zuführungsleitung 6 für die Komponente **K2** mehrere Austrittsöffnungen 8 der Zuführungsleitung 6 auf. Dadurch entsteht beim Austritt aus der Austrittsöffnung 7 der Mischvorrichtung ein Mischbild, wie es in Figur 1 b dargestellt ist, bei welchem mehreren Stränge der Komponente **K2** von der Komponente **K1** umhüllt sind.

Die Figur 2c zeigt schematisch einen Querschnitt entlang der Längsachse z durch eine Mischvorrichtung 3", welche zu einem Mischbild, wie in Figur 1c gezeigt, führt. Die Mischvorrichtung 3" weist eine Zuführungsleitung 4 für die Komponente **K1,** auf, die rohrförmig einen Innenraum 9 begrenzt, in welchem die Komponente **K1** zugeführt wird. In diesen Innenraum 9 ragt eine Zuführungsleitung 6 für die Komponente **K2** hinein. Bei der Applikation, bzw. der Vermischung, werden die Komponente **K1** durch die Zuführungsleitung 4 und die Komponente **K2** durch die Zuführungsleitung 6 gepresst. Nach dem Passieren der Austrittsöffnung 8 der Zuführungsleitung für Komponente **K2** sind die zwei Komponenten **K1** und **K2** in Flussrichtung abwärts miteinander in Kontakt und werden durch mehrere Statikmischer-Elemente 11, welche in einem Statikmischer 10 angeordnet sind, miteinander vermischt. Durch jedes Statikmischer-Element 11 wird der Mischstrom gefaltet, und es entsteht nach dem Austritt aus der Austrittsöffnung 12 des Statikmischers ein schichtenartiges Mischbild im Mischbereich 2. Dadurch, dass der Statikmischer 10 zentrisch in der Zuführungsleitung für die Komponente **K1** angeordnet ist, umfliesst ein Teil der Komponente **K1** den Statikmischer mantelartig und umhüllt nach dem Passieren der Austrittsöffnung 12 des Statikmischers 10 flussabwärts den durch den Statikmischer vermischten Strang 2 aus Komponente **K1** und **K2.** Dadurch entsteht beim Austritt aus der Austrittsöffnung 7 der Mischvorrichtung ein Mischbild, wie es in Figur 1c dargestellt ist.

Die Figur 2d zeigt schematisch einen Querschnitt entlang der Längsachse z durch eine Mischvorrichtung 3''', welche zu einem Mischbild, wie in Figur 1d gezeigt, führt. Die Mischvorrichtung 3''' ist eine Variante der Mischvorrichtung 3", wie sie in Figur 2c diskutiert wurde. Im Gegensatz zur Ausführungsform der Figur 2c wird hier der gesamte Strom der Komponente **K1** mit der Komponente **K2** über mehrere Statikmischer-Elemente 11 vermischt. Dadurch entsteht beim Austritt aus der Austrittsöffnung 7 der Mischvorrichtung ein Mischbild, wie es in Figur 1d dargestellt ist. Zum gleichen Ergebnis gelangt man prinzipiell auch, wenn man an die Austrittsöffnung 7 einer Mischvorrichtung 3, wie sie in Figur 2a gezeigt wurde, einen Statikmischer gemäss dem Stand der Technik anbringt.

Die Figur 2f zeigt schematisch einen Querschnitt entlang der Längsachse z durch eine Mischvorrichtung 3"", welche zu einem Mischbild mit Querschnitt gemäss Figur 1a, bzw. Figur 1f, führt. Die Mischvorrichtung 3'''' weist eine Zuführungsleitung 4 für die Komponente **K1,** auf, die rohrförmig einen Innenraum 9 begrenzt, in welchem die Komponente **K1** zugeführt wird. In diesen Innenraum 9 der Breite $d_B$ ragt eine Zuführungsleitung 6 für die Komponente **K2** hinein. In dieser Ausführungsform ist diese Zuführungsleitung drehbar als Teil eines Drehelements 13 der Breite $d_T$ ausgestaltet. Die Zuführungsleitung endet seitlich am Drehelement 13. In der hier gezeigten Ausführungsform stellt das Endstück 14, welche im Detail in Figur 2f' gezeigt ist, das Ende der Zuführungsleitung 6 der Komponente **K2** dar. Das Endstück 14 weist eine Bohrung 15 auf, welche somit Zuleitung mit der Austrittsöffnung 8 verbindet. Das Endstück wird über ein Gewinde 16 mit dem Rest der Zuführungsleitung 6 verbunden und mittels Arretiermutter 17 fixiert. Durch das Drehen des Drehelementes 13, und somit der Zuführungsleitung 6, beschreibt die Austrittsöffnung 8 im Innern des Innenraumes 9 eine kreisförmige Bewegung. Bei der Applikation, bzw. beim Vermischen, werden gleichzeitig die Komponenten **K1** und **K2** gefördert, und es wird ein spiralförmiger Strang von Komponente **K2,** welcher von Komponente **K1** umhüllt wird, gebildet. Dadurch entsteht beim Austritt aus der Austrittsöffnung 7 der Mischvorrichtung ein Mischbild, wie es in Figur 1a, bzw. in Figur 1f, dargestellt ist.

Es ist dem Fachmann klar, dass die hier gezeigten Ausführungsformen lediglich illustrativ und keineswegs als limitierend zu betrachten sind. Es sind selbstverständlich unterschiedliche Varianten denkbar. Insbesondere kann durch

die Verwendung einer grösseren Anzahl an Statikmischer-Elementen 11 oder durch die Verwendung von dynamischen Mischelementen oder dynamischen Mischern die Vermischung der zwei Komponenten optimiert werden, und es können quasi-homogene oder homogene Vermischungen der beiden Komponenten erhalten werden.

**Bezugszeichenliste**

| | |
|---|---|
| 1 | Raupe |
| 2 | Mischbereich |
| 3, 3', 3", 3''', 3'''' | Mischvorrichtung |
| 4 | Mischeraussenwand |
| 5 | Innenraum der Zuführungsleitung für Komponente **K2** |
| 6 | Zuführungsleitung für Komponente **K2** |
| 7 | Austrittsöffnung der Mischvorrichtung |
| 8 | Austrittsöffnung der Zuführungsleitung für Komponente **K2** |
| 9 | Innenraum der Zuführungsleitung für Komponente **K1** |
| 10 | Statikmischer |
| 11 | Statikmischer-Element |
| 12 | Austrittsöffnung des Statikmischers |
| 13 | Drehelement |
| 14 | Endstück der Zuleitung der Komponente **K2** |
| 15 | Bohrung durch Endstück 14 |
| 16 | Gewinde in Endstück 14 |
| 17 | Arretiermutter |

**Beispiele**

Beschreibung der Messmethoden

**Infrarotspektren** wurden auf einem FT-IR Gerät 1600 von Perkin-Elmer gemessen (horizontale ATR-Messeinheit mit ZnSe-Kristall). Die Proben wurden unverdünnt als Filme aufgetragen. Die Absorptionsbanden sind in Wellenzahlen ($cm^{-1}$) angegeben (Messfenster: 4000-650 $cm^{-1}$).

Der **Amingehalt** der hergestellten Dialdimine, das heisst der Gehalt an geschützten Aminogruppen in Form von Aldiminogruppen, wurde titrimetrisch bestimmt (mit 0.1N $HClO_4$ in Eisessig, gegen Kristallviolett) und ist angegeben in mmol N/g.

a) Herstellung von Dialdiminen der Formel (IV)

**Polyaldimin *A1***

In einem Rundkolben wurden unter Stickstoffatmosphäre 55.0 g (0.19 mol) destilliertes 2,2-Dimethyl-3-lauroyloxy-propanal vorgelegt. Unter kräftigem Rühren wurden aus einem Eintropftrichter 13.7 g (0.18 mol N) 3,6-Dioxaoctan-1,8-diamin (Jeffamine® XTJ-504, Huntsman; Amingehalt 13.34 mmol N/g) langsam zugegeben, wobei sich die Mischung erwärmte. Danach wurden die flüchtigen Bestandteile im Vakuum entfernt (10 mbar, 80 °C). Ausbeute: 65.1 g eines klaren, farblosen Öls mit einem Amingehalt von 2.81 mmol N/g.
IR: 2954, 2920, 2852, 1736 (C=O), 1668 (C=N), 1466, 1419, 1394, 1373, 1366, 1351, 1340sh, 1298sh, 1282sh, 1248, 1232, 1156, 1114, 1057, 1020, 998,932,917sh,876sh,837,791,767,722.

**Dialdimin *A2***

In einem Rundkolben wurden unter Stickstoffatmosphäre 74.3 g (0.26 mol) destilliertes 2,2-Dimethyl-3-lauroyloxy-propanal vorgelegt. Unter kräftigem Rühren wurden aus einem Eintropftrichter 30.0 g (0.25 mol N) Polyetherdiamin (Polyoxypropylen-Diamin mit einem mittleren Molekulargewicht von ca. 240 g/mol; Jeffamine® D-230, Huntsman; Amingehalt 8.32 mmol N/g) langsam zugegeben, wobei sich die Mischung erwärmte und zunehmend eintrübte. Danach wurden die flüchtigen Bestandteile im Vakuum entfernt (10 mbar, 80 °C). Ausbeute: 99.3 g eines klaren, blassgelben Öls mit einem Amingehalt von 2.53 mmol N/g.

b) Herstellung von Polyurethan-Emulsionen

**Emulsion *E1***

Eine Emulsion enthaltend ein Dialdimin der Formel (I) mit einem mittlerem Molekulargewicht von ca. 11'000 wurde folgendermassen hergestellt: In einem Vakuummischer wurden 72.7 g Polyurethanpolymer ***P1,*** dessen Herstellung nachfolgend beschrieben ist, 17.3 g Dialdimin ***A1,*** 0.3 g Salicylsäure-Lösung (5 Gewichts-% in Dioctyladipat) und 90.0 g Polyethylenglykol-dibutylether (Polyglycol BB 300, Clariant; mittleres Molekulargewicht 300) homogen vermischt und auf 60 °C erwärmt. Dazu wurden 76.2 g Wasser eingerührt und die Mischung bei 60 °C während 20 Minuten gerührt. Es wurde eine milchweisse, dünnflüssige Emulsion mit einem Wassergehalt von 29.5 Gewichts-% erhalten.

Das Polyurethanpolymer ***P1*** wurde wie folgt hergestellt:

4000 g Polyoxypropylen-Diol (Acclaim® 4200 N, Bayer; OH-Zahl 28.5 mg KOH/g) und 520 g 4,4'-Methylendiphenyldiisocyanat (MDI; Desmodur® 44 MC L, Bayer) wurden bei 80 °C zu einem Isocyanatgruppen-terminierten Polyurethanpolymeren mit einem titrimetrisch bestimmten Gehalt an freien Isocyanatgruppen von 1.86 Gewichts-% umgesetzt.

**Emulsion *E2***

Eine Emulsion enthaltend ein ionische Gruppen aufweisendes organisches Polymer mit einem mittlerem Molekulargewicht von ca. 20'000 wurde nach bekanntem Verfahren durch Polyaddition von Isophorondiisocyanat (IPDI; Vestanat® IPDI, Degussa) mit Polyol Caradol® ED56-11 (Shell), Aminoethylethanolamin und 2,2-Bis-(hydroxymethyl)-propionsäure in N-Methylpyrrolidon (NMP), anschliessender Neutralisation mit Triethylamin und Zugabe von Wasser bis zu einem Wassergehalt von 29.5 Gewichts-% hergestellt. Es wurde eine milchige Emulsion erhalten.

**Emulsion *E3***

Es wurde versucht, eine der Emulsion ***E2*** analoge, aber NMP-freie Emulsion enthaltend ein ionische Gruppen aufweisendes organisches Polymer herzustellen, indem das N-Methylpyrrolidon in der für die Emulsion ***E2*** beschriebenen Prozedur durch Diethylenglykol-dimethylether (Clariant), welches ein üblicher NMP-Ersatz darstellt, ersetzt wurde. Bei der Zugabe des Aminoethylethanolamins gelierte jedoch das Polymer und liess sich nicht mehr emulgieren.

c) Herstellung von Beschleunigerkomponenten **K2**

**Beschleunigerkomponente *BK1***

In einem Vakuummischer wurden 85 g der Emulsion ***E1*** vorgelegt und mit 1 g techn. Natrium-dodecylbenzolsulfonat (Rhodacal® DS-10, Rhodia), 1 g Natrium-tallat (Dresinate® TX, Eastman), 0.5 g Triethylamin, 7.5 g Polyethylenglykol-dibutylether (Polyglycol BB 300, Clariant; mittleres Molekulargewicht 300), 5 g hydrophiler pyrogener Kieselsäure (Aerosil® 200, Degussa) und 5 g hydrophober pyrogener Kieselsäure (Aerosil® R972, Degussa) zu einer feincremigen Paste vermischt.

**Beschleunigerkomponente *BK2***

In einem Vakuummischer wurden 85 g der Emulsion ***E2*** vorgelegt und mit 10 g hydrophiler pyrogener Kieselsäure (Aerosil® 200, Degussa) und 5 g Kreide (Omya® 5 GU, Omya) zu einer feincremigen Paste vermischt.

**Beschleunigerkomponente *BK3***

In einem Vakuummischer wurden 83.5 g Dialdimin ***A2,*** 6.2 g Wasser, 10.0 g hydrophile pyrogene Kieselsäure (Aerosil® 200, Degussa) und 0.3 g Salicylsäure-Lösung (5 Gewichts-% in Dioctyladipat) zu einer homogenen Paste vermischt.

d) Herstellung einer einkomponentigen Polyurethan-Zusammensetzuna (entsprechend einer Komponente **K1**)

**Zusammensetzung *Z1***

In einem Vakuummischer wurden 180 g Polyurethanpolymer ***P2,*** dessen Herstellung nachfolgend beschrieben ist, 97 g Diisodecylphthalat (DIDP; Palatinol® Z, BASF), 18 g hydrophobe pyrogene Kieselsäure (Aerosil® R972, Degussa), 54 g Russ, 90 g calciniertes Kaolin, 1.3 g 3-Glycidoxypropyltrimethoxysilan (Silquest® A-187, GE Advanced Materials), 0.9 g Desmodur® CD (Bayer) und 9 g einer Lösung von 10 Gewichts-% 2,2'-Dimorpholinodiethylether (DABCO® DMDEE Catalyst, Air Products) und 2 Gewichts-% Dibutylzinndilaurat in DIDP bei 40 °C zu einer homogenen Paste vermischt, die Mischung in innenlackierte Aluminiumkartuschen abgefüllt und diese luftdicht verschlossen. Die Zusammensetzung wies einen Gehalt an Isocyanatgruppen von 0.20 mmol NCO/g und eine Dichte von 1.26 g/cm$^3$ auf.

Das Polyurethanpolymer ***P2*** wurde wie folgt hergestellt:

1300 g Polyoxypropylen-Diol (Acclaim® 4200 N, Bayer; OH-Zahl 28.5 mg KOH/g), 2600 g Polyoxypropylenpolyoxyethylen-Triol (Caradol® MD34-02, Shell; OH-Zahl 35.0 mg KOH/g), 600 g 4,4'-Methylendiphenyldiisocyanat (MDI; Desmodur® 44 MC L, Bayer) und 500 g Diisodecylphthalat (DIDP; Palatinol® Z, BASF) wurden bei 80 °C zu einem Isocyanatgruppen-terminierten Polyurethanpolymeren mit einem Gehalt an freien Isocyanatgruppen von 2.05 Gewichts-% umgesetzt.

e) Herstellung und Prüfung von zweikomponentigen Polyurethan-Klebstoffen (bestehend aus Komponenten **K1** und **K2**)

**Beispiel 1 und Vergleichsbeispiele 2 bis 3**

Für jedes Beispiel wurden die jeweiligen Komponenten **K1** und **K2** gemäss Tabelle 1 im angegebenen Volumenverhältnis im Wesentlichen homogen vermischt und hinsichtlich ihres Aushärteverhaltens untersucht.

Das Mischen der beiden Komponenten **K1** und **K2** erfolgte kontinuierlich während der Applikation, indem der Polyurethan-Klebstoff mittels einer Zweikomponentenpistole mit aufgesetztem Statikmischer vom Typ Sulzer Quadro® (erhältlich von Sulzer Chemtech) mit 24 Mischelementen appliziert wurde.

Zur Untersuchung des Aushärteverhaltens wurden die zweikomponentigen Polyurethan-Klebstoffe einerseits auf Frühfestigkeit und andererseits auf Schneidbarkeit nach der Applikation geprüft.

Zur Prüfung der **Frühfestigkeit** wurde die Zugscherfestigkeit mittels folgender Methode gemessen: Für jede Messung wurden jeweils 2 Glasplättchen von 6 mm Dicke, 25 mm Breite und 75 mm Länge (Floatglas; Firma Rocholl, Schönbrunn, Deutschland) mit Sika® Aktivator (erhältlich bei Sika Schweiz AG) vorbehandelt. Nach einer Ablüftezeit von 10 Minuten wurden die Plättchen mit Hilfe einer passenden PTFE-Form im vertikalen Abstand von 4 mm so gegeneinander angeordnet, dass sie an den Kopfenden um 12 mm überlappten. Der Überlappungsbereich zwischen den Plättchen wurde mit Klebstoff befüllt, wobei dieser auf die aktivierten Seiten der Plättchen zu liegen kam. Die so gegeneinander verklebten Plättchen wurden bei 23 °C und 50% relativer Luftfeuchtigkeit gelagert, nach 2, bzw. 4, bzw. 24, Stunden mit Hilfe einer Zugprüfmaschine (Zwick) bei einer konstanten Querjochgeschwindigkeit von 20 mm/min nach DIN EN 1465 bis zum Bruch auseinander gezogen und die Bruchkraft in MPa (N/mm$^2$) gemessen. Die angegebenen Werte sind Mittelwerte aus drei Messungen.

Zur Bestimmung der **Schneidbarkeit** wurde der Klebstoff in Form einer Dreiecksraupe von ca. 1 cm Grundfläche auf eine LDPE-Folie aufgetragen, die Raupe bei 23 °C und 50% relativer Luftfeuchtigkeit während 2 Stunden belassen und dann mit einem Cutter in Längsrichtung mittig durchschnitten. Der Klebstoff wurde dann als schneidbar bewertet, wenn die Klinge des Cutters beim Schnitt durch die Raupe unverschmutzt blieb, d.h. der Klebstoff soweit vernetzt war, dass er beim Schneiden keine Rückstände von unvernetztem Material hinterliess.

Die Ergebnisse der vorgenommenen Prüfungen sind in der Tabelle 1 aufgeführt.

Tabelle 1: Zusammensetzung der zweikomponentigen Polyurethan-Klebstoffe des Beispiels 1 und der Vergleichsbeispiele 2 bis 3.

| Beispiel | **1** | **2 (Vergleich)** | **3 (Vergleich)** |
|---|---|---|---|
| Komponente **K1** | ***Z1*** | ***Z1*** | ***Z1*** |
| Komponente **K2** | ***BK1*** | ***BK2*** | ***BK3*** |
| Volumenverhältnis **K1/K2** | 98/2 | 98/2 | 95/5 |
| Frühfestigkeit nach 2h (MPa) | >5 | >5 | 0.3 |
| Frühfestigkeit nach 4h (MPa) | >5 | >5 | 0.9 |
| Frühfestigkeit nach 24h (MPa) | >5 | >5 | 3.6 |
| Schneidbarkeit nach 2h | ja | ja | (ja)[a] |

(fortgesetzt)

| Beispiel | 1 | 2 (Vergleich) | 3 (Vergleich) |
|---|---|---|---|
| NMP-haltig | nein | ja | nein |
| [a] sehr weich. | | | |

**Beispiel 4 und Vergleichsbeispiele 5 bis 6**

Für jedes Beispiel wurden die jeweiligen Komponenten **K1** und **K2** gemäss Tabelle 2 im angegebenen Volumenverhältnis im Wesentlichen schichtenartig vermischt und hinsichtlich ihres Aushärteverhaltens untersucht.

Das Mischen der beiden Komponenten **K1** und **K2** erfolgte kontinuierlich während der Applikation, indem der Polyurethan-Klebstoff mittels einer Zweikomponentenpistole mit aufgesetztem Statikmischer mit 10 Mischelementen (Typ TAH-510, Firma GLT, Deutschland) appliziert wurde.

Zur Untersuchung des Aushärteverhaltens wurden die zweikomponentigen Polyurethan-Klebstoffe einerseits auf Frühfestigkeit und andererseits auf Schneidbarkeit nach der Applikation geprüft wie bei Beispiel 1 beschrieben. Die Ergebnisse der vorgenommenen Prüfungen sind in der Tabelle 2 aufgeführt.

Tabelle 2: Zusammensetzung der zweikomponentigen Polyurethan-Klebstoffe des Beispiels 4 und der Vergleichsbeispiele 5 bis 6.

| Beispiel | **4** | **5 (Vergleich)** | **6 (Vergleich)** |
|---|---|---|---|
| Komponente **K1** | ***Z1*** | ***Z1*** | ***Z1*** |
| Komponente **K2** | ***BK1*** | ***BK2*** | ***BK3*** |
| Volumenverhältnis **K1/K2** | 98/2 | 98/2 | 95/5 |
| Frühfestigkeit nach 2h (MPa) | 1.6 | >5 | n.m.[a] |
| Frühfestigkeit nach 4h (MPa) | 1.9 | >5 | 0.7 |
| Frühfestigkeit nach 24h (MPa) | 2.7 | >5 | 2.0 |
| Schneidbarkeit nach 2h | ja | ja | nein |
| NMP-haltig | nein | ja | nein |
| [a] nicht messbar. | | | |

Der Vergleich des erfindungsgemässen Beispiels **1** mit dem Vergleichsbeispiel **2,** welches auf einer NMP-haltigen Beschleunigerkomponente **BK2** entsprechend dem Stand der Technik basiert, zeigt, dass bei der im Wesentlichen homogenen Vermischung eine exzellente Frühfestigkeit erhalten wird. Eine der Beschleunigerkomponente ***BK2*** entsprechende NMP-freie Beschleunigerkomponenten war nicht herstellbar, da bereits (wie schon vorher beschrieben) bei der Herstellung der entsprechenden Emulsion **E3** eine Gelierung auftrat.

Weiterhin zeigen die Ergebnisse aus den Tabellen 1 und 2 auf, dass die erfindungsgemässen Beispiele **1** und **4** gegenüber den Vergleichsbeispielen **3** und **6,** welche auf einer Beschleunigerkomponente ***BK3*** entsprechend dem Stand der Technik basieren, eine stark verbesserte Frühfestigkeit und eine bessere Schneidbarkeit aufweisen.

## Patentansprüche

1. Dialdimin der Formel (I)

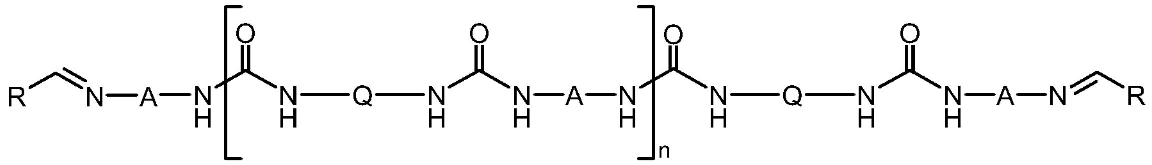

(I)

wobei R für den Rest eines Aldehyds **ALD** nach Entfernung einer Aldehyd-Gruppe steht;
A für den Rest eines Diamins **DA** mit zwei primären aliphatischen Aminogruppen nach Entfernung der beiden primären aliphatischen Aminogruppen steht;
Q für den Rest eines Diisocyanates **DI** nach Entfernung beider Isocyanatgruppen steht;
n für 0 oder für eine ganze Zahl von 1 bis 15 steht; und

wobei A und R keine Gruppierungen aufweisen, welche in Abwesenheit von Wasser mit Isocyanatgruppen reaktionsfähig sind.

**2.** Dialdimin gemäss Anspruch 1, **dadurch gekennzeichnet, dass** R für den Rest der Formel (II) steht

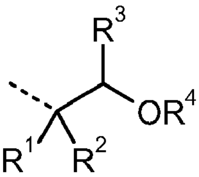

(II)

wobei $R^1$ und $R^2$
entweder unabhängig voneinander jeweils für einen einwertigen Kohlenwasserstoffrest mit 1 bis 12 C-Atomen stehen;
oder zusammen für einen zweiwertigen Kohlenwasserstoffrest mit 4 bis 20 C-Atomen stehen, welcher Teil eines, gegebenenfalls substituierten, carbocyclischen Rings mit 5 bis 8, bevorzugt mit 6, C-Atomen ist;
$R^3$ für ein Wasserstoffatom oder für eine Alkyl- oder Arylalkylgruppe steht; $R^4$
für einen, gegebenenfalls Heteroatome enthaltenden, Kohlenwasserstoffrest mit 1 bis 30 C-Atomen steht;
oder für einen Rest der Formel (III') steht,

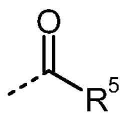

(III')

wobei $R^5$
für einen linearen oder verzweigten Alkylrest mit 1 bis 30 C-Atomen, gegebenenfalls mit cyclischen Anteilen und gegebenenfalls mit mindestens einem Heteroatom, insbesondere Sauerstoff in Form von Ether- oder Carbonyl- oder Ester-Gruppen, steht; oder für einen einfach oder mehrfach ungesättigten linearen oder verzweigten Kohlenwasserstoffrest mit 1 bis 30 C-Atomen steht.

**3.** Dialdimin gemäss Anspruch 2, **dadurch gekennzeichnet, dass** $R^4$
für einen, gegebenenfalls Heteroatome enthaltenden, Kohlenwasserstoffrest mit 11 bis 30 C-Atomen steht;
oder für einen Rest der Formel (III') steht,

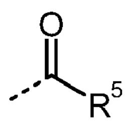

(III')

wobei $R^5$

für einen linearen oder verzweigten Alkylrest mit 11 bis 30 C-Atomen, gegebenenfalls mit cyclischen Anteilen und gegebenenfalls mit mindestens einem Heteroatom, insbesondere Sauerstoff in Form von Ether- oder Carbonyl- oder Ester-Gruppen, steht; oder für einen einfach oder mehrfach ungesättigten linearen oder verzweigten Kohlenwasserstoffrest mit 11 bis 30 C-Atomen steht.

**4.** Dialdimin gemäss einem der Ansprüche 1 bis 3, **dadurch gekennzeichnet, dass** die Reste A und R keine Hydroxylgruppen, keine primären oder sekundären Aminogruppen, keine Mercaptogruppen und keine anderen Gruppen mit aktivem Wasserstoff aufweisen.

**5.** Dialdimin gemäss einem der vorhergehenden Ansprüche, **dadurch gekennzeichnet, dass** das Diisocyanat **DI** ein Isocyanatgruppen aufweisendes Polyurethanpolymer **P** ist.

**6.** Dialdimin gemäss Anspruch 5, **dadurch gekennzeichnet, dass** das Isocyanatgruppen aufweisende Polyurethanpolymer **P** aus der Umsetzung von mindestens einem Diol mit mindestens einem monomeren Diisocyanat hergestellt wird.

**7.** Dialdimin gemäss Anspruch 6, **dadurch gekennzeichnet, dass** das monomere Diisocyanat ein aromatisches Diisocyanat, insbesondere 2,4- oder 2,6-Toluylendiisocyanat (TDI) oder 4,4'-, 2,4'- oder 2,2'-Diphenylmethandiisocyanat (MDI), ist.

**8.** Dialdimin gemäss Anspruch 6 oder 7, **dadurch gekennzeichnet, dass** das Diol ein Polyoxyalkylendiol, insbesondere ein Polyoxyethylendiol, ein Polyoxypropylendiol oder ein Polyoxypropylenpolyoxyethylendiol, ist.

**9.** Dialdimin gemäss einem der vorhergehenden Ansprüche, **dadurch gekennzeichnet, dass** das Dialdimin der Formel (I) ein Molekulargewicht von 1'000 bis 30'000 g/mol, insbesondere von 2'000 bis 30'000 g/mol, bevorzugt von 4'000 bis 30'000 g/mol, meist bevorzugt von 6'000 bis 20'000 g/mol, aufweist.

**10.** Dialdimin gemäss einem der vorhergehenden Ansprüche, **dadurch gekennzeichnet, dass** das Diamin **DA** ein Ethergruppen-haltiges aliphatisches Diamin, insbesondere ein Ethergruppen-haltiges aliphatisches Diamin, welches ausgewählt ist aus der Gruppe bestehend aus Bis-(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin und höheren Oligomeren dieser Diamine, sowie Polyoxyalkylen-Diaminen der Formel (V'), ist,

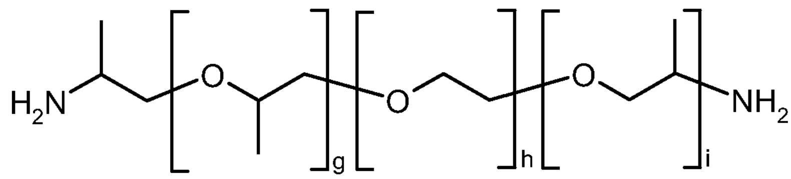
(V')

wobei g, h und i jeweils für 0 oder eine ganze Zahl von 1 bis 40 stehen mit der der Massgabe, dass die Summe von g, h und i ≥ 1 ist.

**11.** Verfahren zur Herstellung eines Dialdimins der Formel (I) gemäss einem der Ansprüche 1 bis 10 umfassend den Schritt der Umsetzung eines Diisocyanates **DI** mit einem Dialdimin der Formel (IV), insbesondere der Formel (IV') oder der Formel (IV"), in Gegenwart von Wasser,

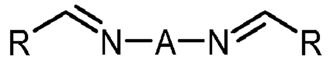
(IV)

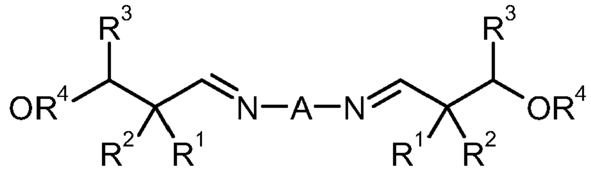

(IV')

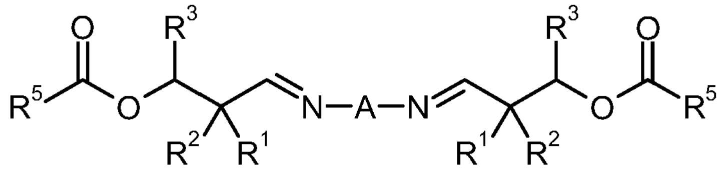

(IV'')

wobei das Diisocyanat **DI** und das Dialdimin der Formel (IV) oder (IV') oder (IV") im molaren Verhältnis von [Diisocyanat **DI]** / [Dialdimin der Formel (IV) oder (IV') oder (IV")] von < 1, insbesondere von 0.9 bis 0.5, bevorzugt von 0.8 bis 0.5, eingesetzt werden und
wobei die Menge des Wassers derart gewählt ist, dass das molare Verhältnis von [Wasser] / [Diisocyanat **DI]** ≥ 2, bevorzugt ≥ 10, ist.

**12.** Aldiminhaltige Emulsion umfassend

a) mindestens ein Dialdimin der Formel (I) gemäss einem der Ansprüche 1 bis 10;
b) Wasser; und
c) gegebenenfalls mindestens ein Tensid.

**13.** Aldiminhaltige Emulsion gemäss Anspruch 12, **dadurch gekennzeichnet, dass** die Emulsion frei von organischen VOC-Lösemitteln, insbesondere frei von N-Alkylpyrrolidonen, ist.

**14.** Verwendung einer aldiminhaltigen Emulsion gemäss Anspruch 12 oder 13 als Härterkomponente oder Beschleunigerkomponente für einen Klebstoff, Dichtstoff oder Beschichtungsstoff, welcher Isocyanatgruppen aufweisende Polymere enthält.

**15.** Zweikomponentige Zusammensetzung bestehend aus zwei Komponenten **K1** und **K2,** wobei
die Komponente **K1** mindestens ein Isocyanatgruppen aufweisendes Polymer, insbesondere mindestens ein Isocyanatgruppen aufweisendes Polyurethanpolymer, enthält oder daraus besteht;
und
die Komponente **K2** ein Dialdimin der Formel (I) gemäss einem der Ansprüche 1 bis 10 oder eine aldiminhaltige Emulsion gemäss Anspruch 12 oder 13 enthält oder daraus besteht.

**16.** Vermischte oder teilvermischte zweikomponentige Zusammensetzung, **dadurch gekennzeichnet, dass** die Komponente **K1** und die Komponente **K2** einer zweikomponentigen Zusammensetzung gemäss Anspruch 15 weitgehend homogen oder inhomogen miteinander vermischt sind, wobei die Vermischung der zwei Komponenten vorzugsweise über einen Statikmischer oder einen dynamischen Mischer erfolgt.

**17.** Verwendung einer zweikomponentigen Zusammensetzung gemäss Anspruch 15 als Klebstoff, Dichtstoff oder Beschichtung oder Belag.

**18.** Verfahren der Verklebung von Substraten **S1** und **S2** umfassend die Schritte

i) Applizieren einer zweikomponentigen Zusammensetzung gemäss Anspruch 15 während des Mischens oder nach dem Mischen der zwei Komponenten **K1** und **K2** auf ein Substrat **S1;**
ii) Kontaktieren der applizierten Zusammensetzung mit einem Substrat **S2;**

oder

i') Applizieren einer zweikomponentigen Zusammensetzung gemäss Anspruch 15 während des Mischens oder

nach dem Mischen der zwei Komponenten **K1** und **K2** auf ein Substrat **S1;**
ii') Applizieren einer zweikomponentigen Zusammensetzung gemäss Anspruch 15 während des Mischens oder nach dem Mischen der zwei Komponenten **K1** und **K2** auf ein Substrat **S2;**
iii') Kontaktieren der auf Substrat **S1** applizierten Zusammensetzung mit der auf Substrat **S2** applizierten Zusammensetzung;

wobei die Substrate **S1** und **S2** gleich oder verschieden voneinander sind.

**19.** Verfahren des Abdichtens umfassend den Schritt

i") Applizieren einer zweikomponentigen Zusammensetzung gemäss Anspruch 15 während des Mischens oder nach dem Mischen der zwei Komponenten **K1** und **K2** zwischen ein Substrat **S1** und ein Substrat **S2;**

wobei die Substrate **S1** und **S2** gleich oder verschieden voneinander sind.

**20.** Verfahren zur Herstellung einer Beschichtung, insbesondere eines Bodenbelages, umfassend den Schritt

i"') Applizieren einer zweikomponentigen Zusammensetzung gemäss Anspruch 15 während des Mischens oder nach dem Mischen der zwei Komponenten **K1** und **K2** auf ein Substrat **S1.**

**21.** Verfahren gemäss Anspruch 18, 19 oder 20, **dadurch gekennzeichnet, dass** das Mischen der zwei Komponenten **K1** und **K2** im Wesentlichen homogen erfolgt.

**22.** Verfahren gemäss Anspruch 18, 19 oder 20, **dadurch gekennzeichnet, dass** das Mischen der zwei Komponenten **K1** und **K2** im Wesentlichen inhomogen erfolgt, insbesondere derart inhomogen, dass ein schichtenartiges, spiralförmiges oder strangförmiges Mischbild gebildet wird.

**23.** Verfahren gemäss einem der Ansprüche 18 bis 22, **dadurch gekennzeichnet, dass** das Substrat **S1** und/oder **S2** ausgewählt ist aus der Gruppe bestehend aus Beton, Zement, Mörtel, Backstein, Ziegel, Gips, Naturstein, Asphalt, Metall, Metalllegierung, Holz, Keramik, Glas, Kunststoff, Pulverbeschichtung, Farbe und Lack.

**24.** Artikel, welcher nach einem Verfahren gemäss einem der Ansprüche 18 bis 23 verklebt, abgedichtet oder beschichtet wurde.

**25.** Artikel gemäss Anspruch 24, **dadurch gekennzeichnet, dass** der Artikel ein Gebäude oder ein Bauwerk des Hoch- oder Tiefbaus, ein industriell gefertigtes Gut oder ein Konsumgut, insbesondere ein Fenster, eine Haushaltsmaschine, oder ein Transportmittel, insbesondere ein Fahrzeug, oder ein Anbauteil eines Fahrzeugs, ist.

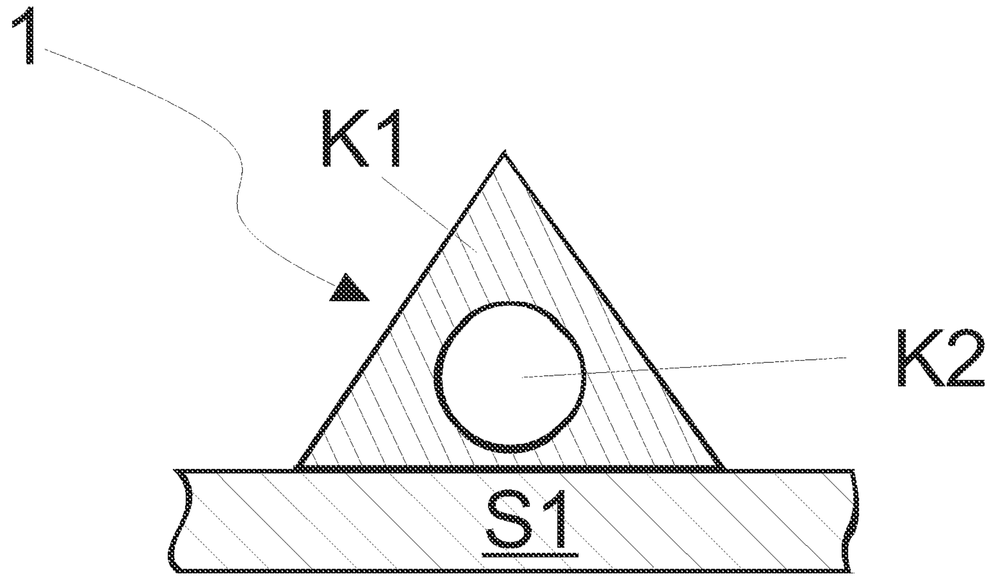


Figur 1 a)

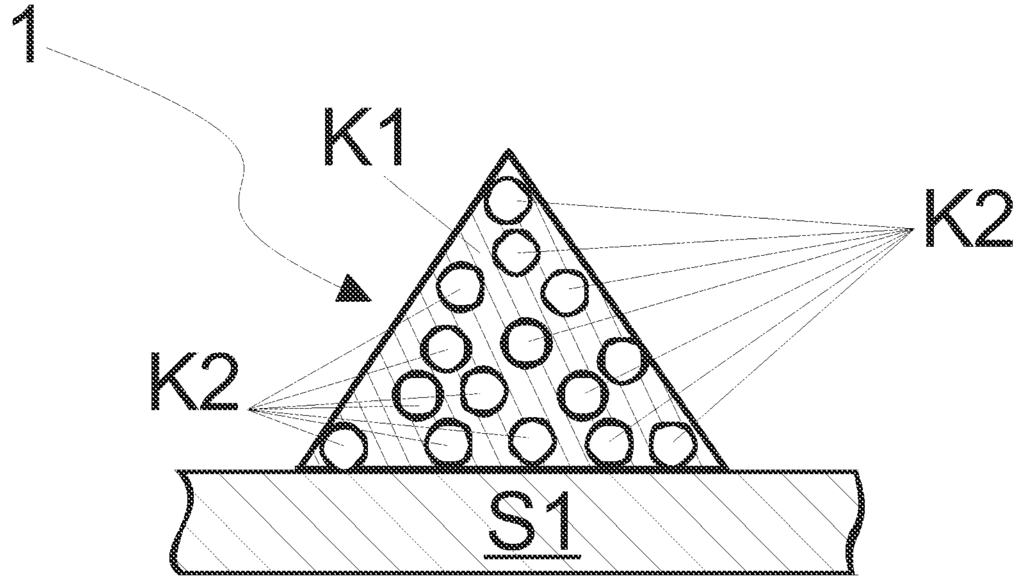


Figur 1 b)

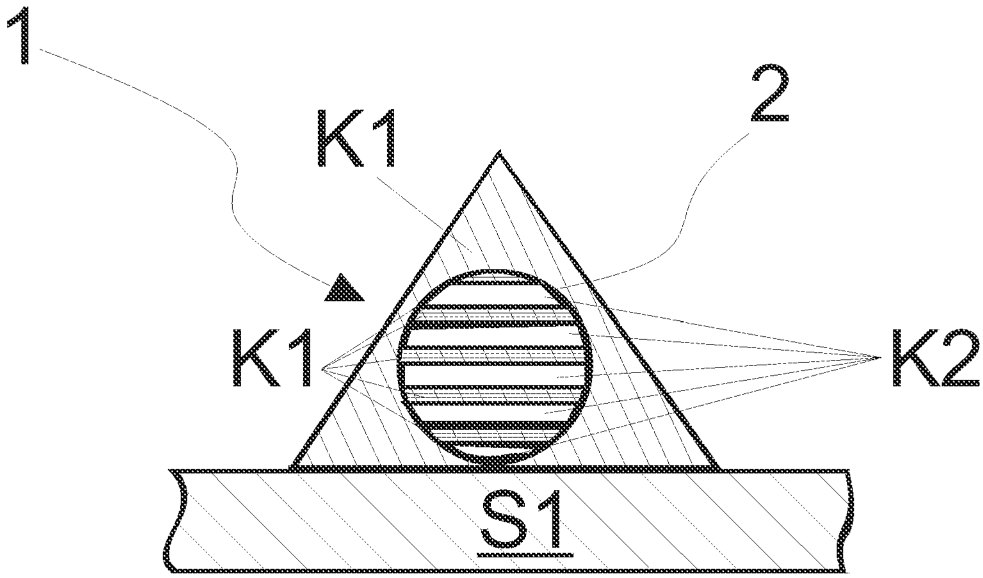


Figur 1 c)

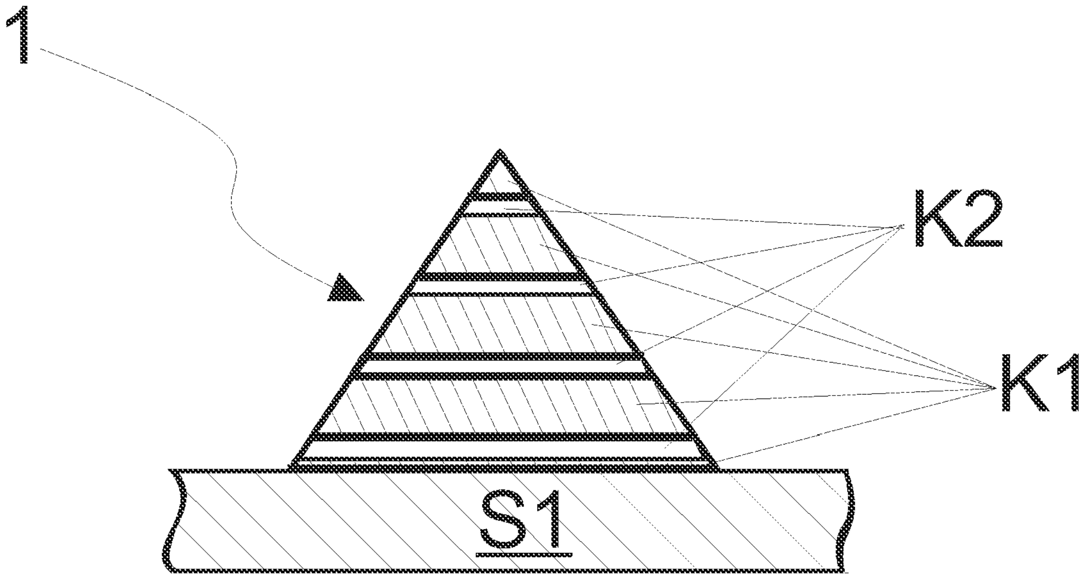


Figur 1 d)

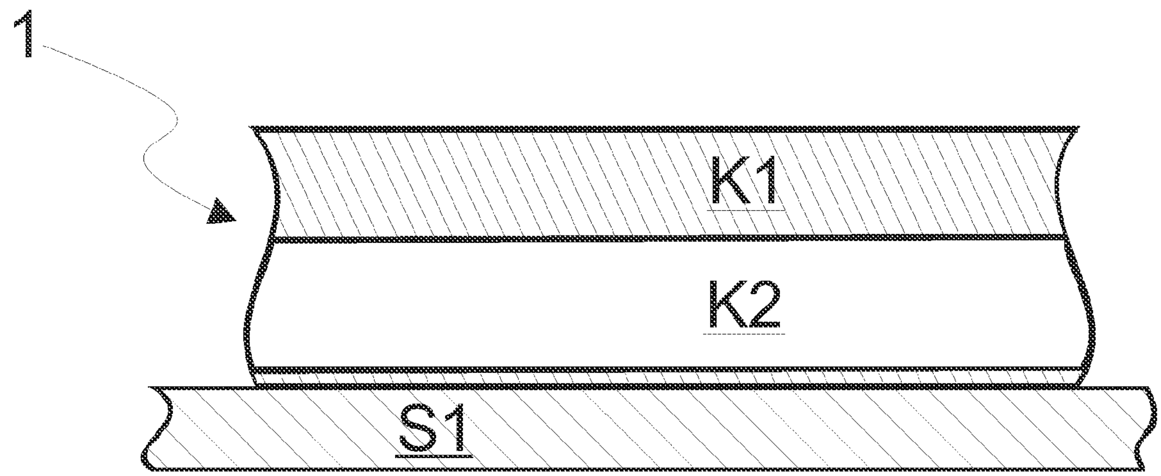


Figur 1 e)

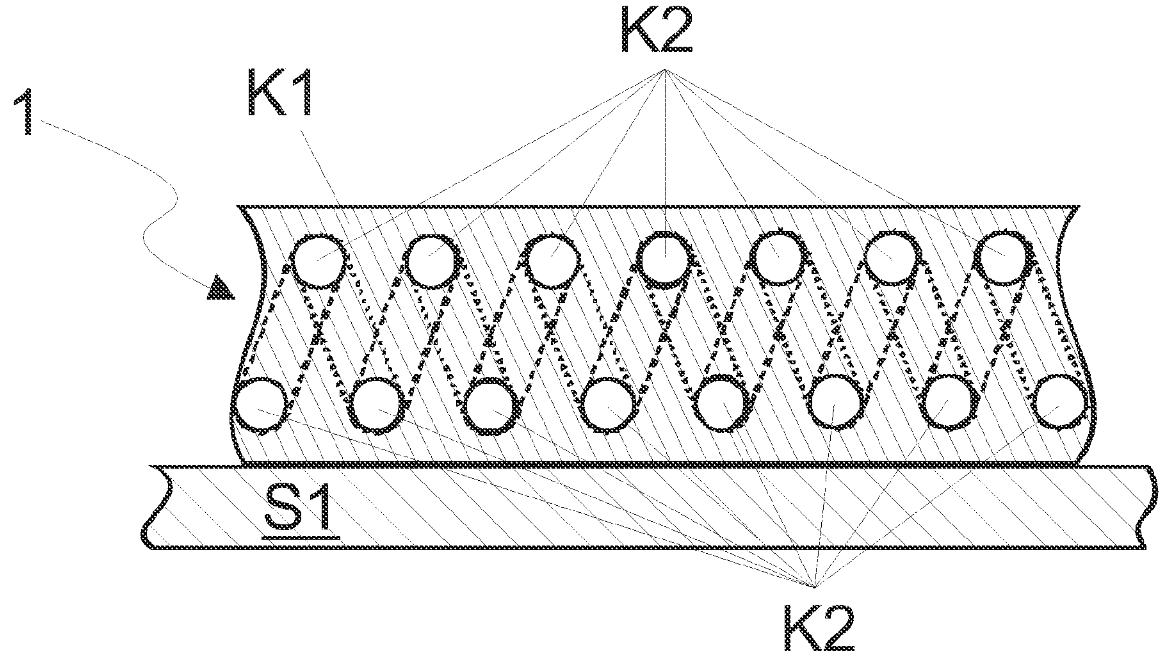


Figur 1 f)

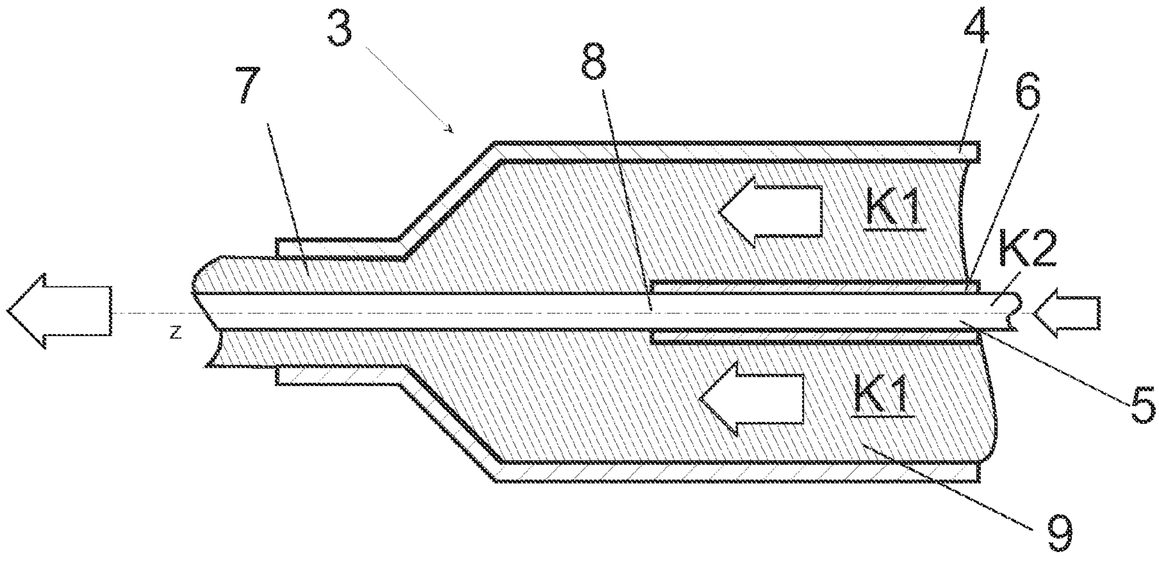


Figur 2a)

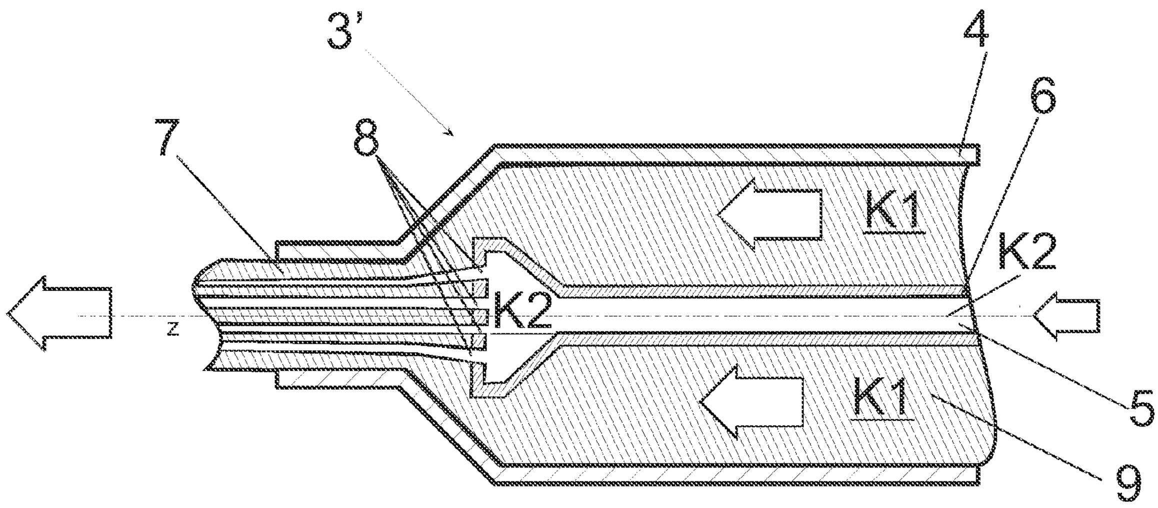


Figur 2b)

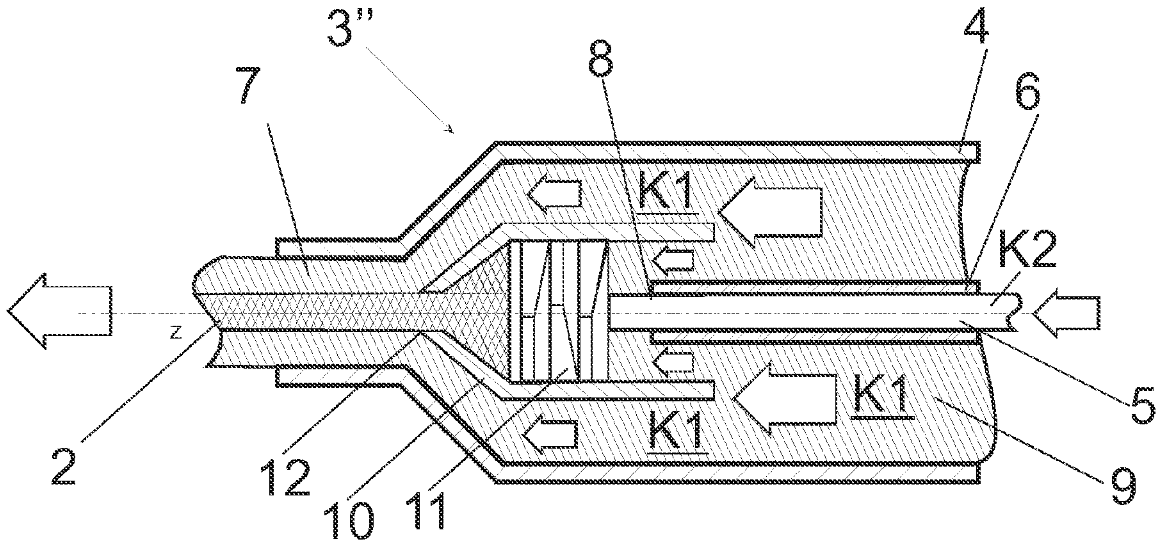


**Figur 2c)**

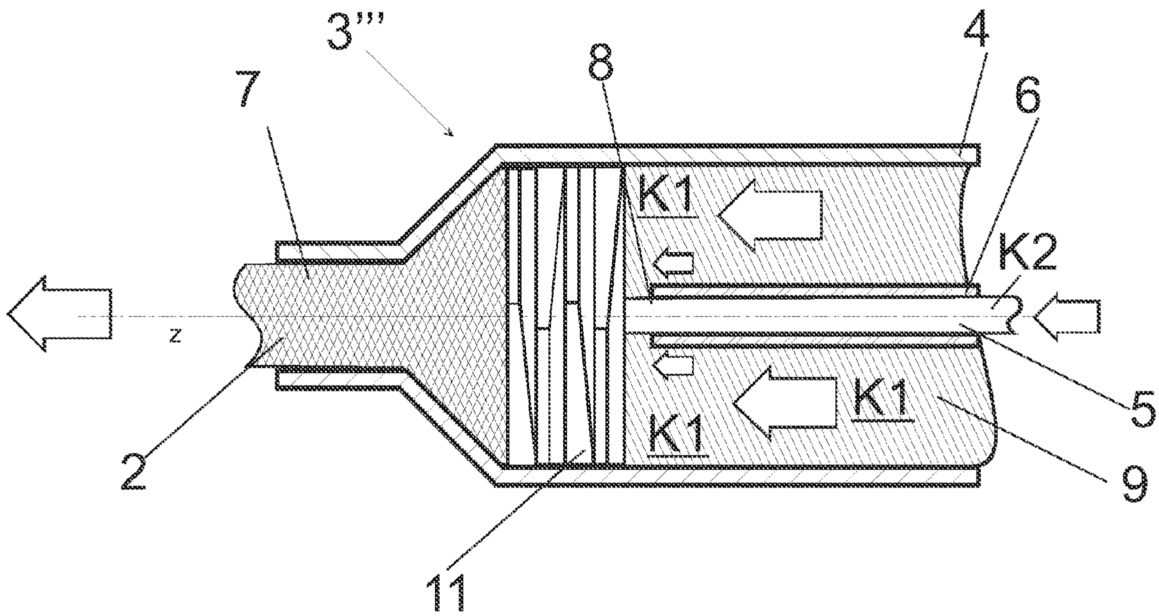


**Figur 2d)**

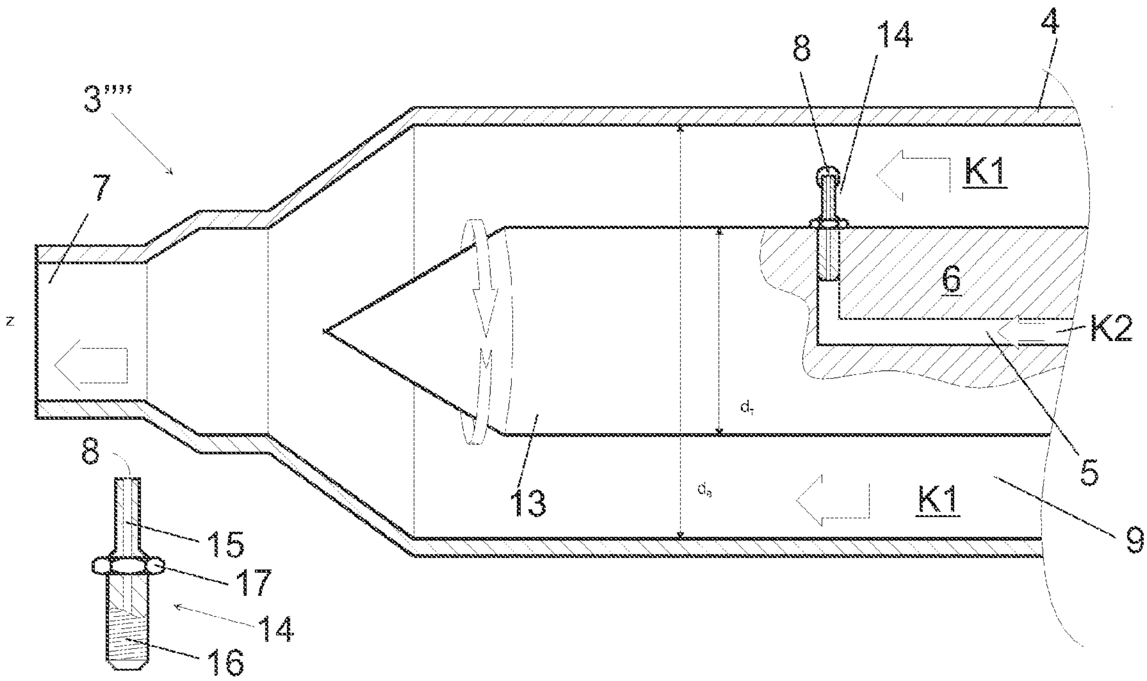


Figur 2 f')

Figur 2 f)

Europäisches Patentamt

# EUROPÄISCHER RECHERCHENBERICHT

Nummer der Anmeldung
EP 07 10 3554

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DATABASE CA [Online] CHEMICAL ABSTRACTS SERVICE, COLUMBUS, OHIO, US; MOTOMIYA, TATSUYA: "Reactive fluorescent brightening agents" XP002476906 gefunden im STN Database accession no. 1966:60393 * Zusammenfassung * & JP 40 029011 B4 (NISSHIN SPINNING CO., LTD.) 23. Dezember 1965 (1965-12-23) ----- | 1 | INV. C07C249/02 C07C251/02 C07C275/00 C08G18/12 |
| X | DATABASE CA [Online] CHEMICAL ABSTRACTS SERVICE, COLUMBUS, OHIO, US; HOENEN, LAMBERT: "4,4'-Disubstituted diphenyl sulfones" XP002476907 Database accession no. 1965:51383 * Zusammenfassung * & US 3 165 510 A (LAMBERT HOENEN ET AL) 12. Januar 1965 (1965-01-12) ----- | 1 | RECHERCHIERTE SACHGEBIETE (IPC) |
| X | DATABASE CA [Online] CHEMICAL ABSTRACTS SERVICE, COLUMBUS, OHIO, US; NAUMOV, YU: "Preparation of 3-amino-4-methoxybenzenesulfonyl fluoride" XP002476908 Database accession no. 1965:51384 * Zusammenfassung * & SU 16 459 A (NAUMOV, YU) 1964 ----- | 1 | C07C C08G |
| A,D | EP 1 524 282 A (SIKA TECHNOLOGY AG [CH]) 20. April 2005 (2005-04-20) * Absätze [0016], [0063] - [0079] * ----- -/-- | 1-25 | |

Der vorliegende Recherchenbericht wurde für alle Patentansprüche erstellt

2

| Recherchenort | Abschlußdatum der Recherche | Prüfer |
|---|---|---|
| München | 5. Mai 2008 | Panday, Narendra |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 07 10 3554

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 03/059978 A (SIKA TECHNOLOGY AG [CH]; BURCKHARDT URS [CH]; STADELMANN URSULA [CH];) 24. Juli 2003 (2003-07-24)<br>* Beispiele A1-A5,1-7 *<br>----- | 1-25 | |
| A | US 4 108 842 A (KONIG EBERHARD ET AL) 22. August 1978 (1978-08-22)<br>* das ganze Dokument *<br>----- | 1-25 | |

RECHERCHIERTE SACHGEBIETE (IPC)

Der vorliegende Recherchenbericht wurde für alle Patentansprüche erstellt

2

| Recherchenort | Abschlußdatum der Recherche | Prüfer |
|---|---|---|
| München | 5. Mai 2008 | Panday, Narendra |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 07 10 3554

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-05-2008

| Im Recherchenbericht angeführtes Patentdokument | Datum der Veröffentlichung | Mitglied(er) der Patentfamilie | Datum der Veröffentlichung |
|---|---|---|---|
| JP 40029011 B4 | | KEINE | |
| US 3165510 A | 12-01-1965 | DE 1164393 B | 05-03-1964 |
| | | GB 973900 A | 28-10-1964 |
| SU 16459 A | | KEINE | |
| EP 1524282 A | 20-04-2005 | BR PI0415367 A | 12-12-2006 |
| | | CA 2551404 A1 | 28-04-2005 |
| | | CN 1882627 A | 20-12-2006 |
| | | WO 2005037885 A1 | 28-04-2005 |
| | | JP 2007509200 T | 12-04-2007 |
| | | US 2007129522 A1 | 07-06-2007 |
| WO 03059978 A | 24-07-2003 | AU 2003235705 A1 | 30-07-2003 |
| | | CA 2473437 A1 | 24-07-2003 |
| | | CN 1610709 A | 27-04-2005 |
| | | JP 2005514503 T | 19-05-2005 |
| | | US 2007004893 A1 | 04-01-2007 |
| | | US 2006052522 A1 | 09-03-2006 |
| US 4108842 A | 22-08-1978 | AT 358283 B | 25-08-1980 |
| | | AU 514144 B2 | 29-01-1981 |
| | | AU 2792677 A | 22-02-1979 |
| | | BE 857854 A1 | 17-02-1978 |
| | | BR 7705457 A | 02-05-1978 |
| | | CA 1114989 A1 | 22-12-1981 |
| | | CS 196218 B2 | 31-03-1980 |
| | | DD 133805 A5 | 24-01-1979 |
| | | DE 2637115 A1 | 23-02-1978 |
| | | ES 461680 A1 | 01-12-1978 |
| | | FR 2362171 A1 | 17-03-1978 |
| | | GB 1545005 A | 25-04-1979 |
| | | HU 178432 B | 28-05-1982 |
| | | IT 1079426 B | 13-05-1985 |
| | | JP 1425441 C | 15-02-1988 |
| | | JP 53024396 A | 07-03-1978 |
| | | JP 62027092 B | 12-06-1987 |
| | | NL 7709107 A | 21-02-1978 |
| | | PL 200320 A1 | 24-04-1978 |
| | | RO 72918 A1 | 04-11-1981 |
| | | SE 7709278 A | 19-02-1978 |
| | | SU 741799 A3 | 15-06-1980 |
| | | YU 198277 A1 | 31-05-1982 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2004013200 A1
- WO 2005037885 A1
- WO 9524556 A1
- EP 1728840 A1
- WO 0232562 A1


### In der Beschreibung aufgeführte Nicht-Patentliteratur


- **HOUBEN-WEYL.** *Methoden der organischen Chemie,* vol. VIII, 516-528